# EUROPÄISCHE PATENTSCHRIFT

(11) **EP 1 261 601 B1**
(45) Veröffentlichungstag und Bekanntmachung des Hinweises auf die Patenterteilung: **29.07.2009**
(21) Anmeldenummer: 00974405.3
(22) Anmeldetag: 17.10.2000
(51) Int. Cl.: C07D 401/04, A61P 19/02, A61P 29/00

(54) **SUBSTITUIERTE INDOLE ZUR MODULIERUNG VON NFKB-AKTIVITÄT**
SUBSTITUTED INDOLES FOR MODULATING NFKB ACTIVITY
INDOLS SUBSTITUES POUR MODULER L'ACTIVITE DU FACTEUR DE TRANSCRIPTION NFKB

(30) Priorität: 26.10.1999 DE 19951360
(43) Veröffentlichungstag der Anmeldung: 04.12.2002
(73) Patentinhaber: Sanofi-Aventis Deutschland GmbH, 65929 Frankfurt am Main (DE)
(72) Erfinder: RITZELER, Olaf, 65812 Bad Soden (DE); STILZ, Hans, Ulrich, 65929 Frankfurt (DE); NEISES, Bernhard, 77652 Offenburg (DE); JAEHNE, Gerhard, 65929 Frankfurt (DE); HABERMANN, Jörg, 65812 Bad Soden (DE)
(86) Internationale Anmeldenummer: PCT/EP2000/010210
(87) Internationale Veröffentlichungsnummer: WO 2001/030774

(56) Entgegenhaltungen:
- WO-A-94/08962
- WO-A-94/12478
- WO-A-99/43654
- US-A- 5 723 485

## Beschreibung

Die Erfindung betrifft neue substituierte Indole, Verfahren zu ihrer Herstellung und Verwendung derselben als Arzneimittel.

In der Patentanmeldung WO 94/12478 werden unter anderem Indolderivate beschrieben, die die Blutplättchen-Aggregation inhibieren.
NFκB ist ein heterodimerer Transkriptionsfaktor, der eine Vielzahl von Gene aktivieren kann, die unter anderen für proinflammatorische Cytokine wie IL-1, IL-2, TNFα oder IL-6 kodieren. NFκB liegt im Cytosol von Zellen komplexiert mit seinem natürlich vorkommenden Inhibitor lκB vor. Die Stimulation von Zellen, beispielsweise durch Cytokine, führt zur Phosphorylierung und anschließenden proteolytischen Abbau von lκB. Dieser proteolytische Abbau führt zur Aktivierung von NFκB, das anschließend in den Kern der Zelle wandert und dort eine Vielzahl von proinflammatorischen Genen aktiviert.
In Erkrankungen wie Rheumatoider Arthritis (bei der Entzündung), Osteoarthritis, Asthma, Herzinfarkt, Alzheimer Erkrankungen oder Atherosklerose ist NFκB über das normale Maß hinaus aktiviert. Die Hemmung von NFκB ist auch in der Krebstherapie von Nutzen, da sie dort zur Verstärkung der Cytostatika Therapie eingesetzt wird. Es konnte gezeigt werden, daß Arzneimittel wie Glucocorticoide, Salicylate oder Goldsalze, die in der Rheumatherapie eingesetzt werden, an verschiedenen Stellen in die NFκBaktivierende Signalkette inhibierend eingreifen oder direkt mit der Transkription der Gene interferieren.
Der erste Schritt in der genannten Signalkaskade ist der Abbau von lκB. Diese Phosphorylierung wird durch die spezifische lκB-Kinase reguliert. Bisher sind keine Inhibitoren bekannt, die spezifisch lκB-Kinase inhibieren.

In dem Bestreben wirksame Verbindungen zur Behandlung von Rheumatoider Arthritis (bei der Entzündung), Osteoarthritis, Asthma, Herzinfarkt, Alzheimer Erkrankungen, Krebserkrankungen (Potenzierung von Cytotoxica-Therapien) oder Atherosklerose zu erhalten, wurde nun gefunden, daß die erfindungsgemäßen Indolderivate starke und sehr spezifische Inhibitoren der lκB-Kinase sind.

Die Erfindung betrifft daher die Verbindung der Formel I 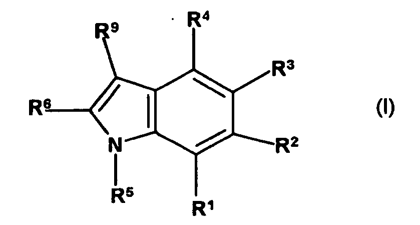 und/oder eine stereoisomere Form der Verbindung der Formel I und/oder ein physiologisch verträgliches Salz der Verbindung der Formel I, wobei
R³ für einen Rest der Formel II steht, 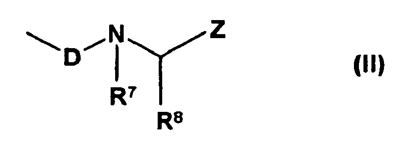 worin
- D für: -C(O)- steht,
- R⁷ für: Wasserstoffatom oder -(C₁-C₄)-Alkyl steht,
- R⁸ für: 1. -(C₁-C₄)-Alkyl, worin Alkyl gerade oder verzweigt ist und ein- oder zweifach unabhängig voneinander substituiert ist durch
   1.1 Heteroaryl mit 5 bis 14 Ringgliedern oder Heterocyclus mit 5 bis 12 Ringgliedern, worin Heteroaryl und Heterocyclus ausgewählt wurden aus der Gruppe Pyrrol, Pyridin, Pyrazin, Furan, Thiophen, Imidazol, Pyrazol, Oxazol, Isoxazol, Thiazol, Isothiazol, Tetrazol, Triazolone, 1,2,3,5-Oxathiadiazol-2-Oxide, Oxadiazolone, Isoxazolone, Oxadiazolidindione, Triazole, welche durch F, -CN, -CF₃ oder -C(O)-O-(C₁-C₄)-Alkyl substituiert sind, 3-Hydroxypyrro-2,4-dione, 5-Oxo-1,2,4-Thiadiazole, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- und cyclohepta-anellierte Derivate dieser Heterocyclen ableiten,
   1.2 -O-R¹⁰,
   1.3 -S(O)ₓ-R¹⁰, worin x die ganze Zahl Null, 1 oder 2 ist,
   1.4 -N(R¹⁰)₂,
   1.5 Rest der Formel 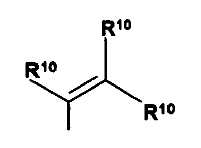 oder
   1.6 Rest der Formel 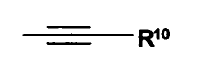 oder
2. für den charakteristischen Rest einer Aminosäure steht aus der Gruppe Glycin, Alanin, Valin, Leucin, Isoleucin, Phenylalanin, Tyrosin, Serin, Tryptophan, Threonin, Cystein, Methionin, Asparagin, Glutamin, Lysin, Histidin, Arginin, Glutaminsäure, Asparaginsäure, 2-Aminoadipinsäure, 2-Aminoisobutter-säure, 2-Aminobuttersäure, 2,3-Diamino-propionsäure, 2,4-Diaminobuttersäure, 1,2,3,4-Tetrahydroisochinolin-1-carbonsäure, 1,2,3,4-Tetrahydroisochinolin-3-carbonsäure, 2-Aminopimelinsäure, Phenylglycin, 3-(2-Thienyl)-alanin, 3-(3-Thienyl)-alanin, 2-(2-Thienyl)-glycin, 2-Amino-heptansäure, Pipecolinsäure, Hydroxylysin, Sarkosin, N-Methylisoleucin, 6-N-Methyllysin, N-Methylvalin, Norvalin, Norleucin, Omithin, allo-Isoleucin, allo-Threonin, allo-Hydroxylysin, 4-Hydroxyprolin, 3-Hydroxyprolin, 3-(2-Naphtyl)-alanin, 3-(1-Naphtyl-alanin), Homophenylalanin, Homocystein, Homocysteinsäure, Homotryptophan, Cysteinsäure, 3-(2-Pyridyl)-alanin, 3-(3-Pyridyl)-alanin, 3-(4-Pyridyl)-alanin, 2-Amino-3-phenylaminopropionsäure, 2-Amino-3-phenylamino-ethylpropionsäure, Phosphinothricin, 4-Fluorphenylalanin, 3-Fluorphenyl-alanin, 2-Fluorphenylalanin, 4-Chlorphenylalanin, 4-Nitrophenylalanin, 4-Aminophenylalanin, Citrullin, Cyclohexylalanin, 5-Flüortryptophan, 5-Methoxytryptophan, Methionin-Sulfon, Methionin-Sulfoxid und -NH-NR¹⁰-C(O)N(R¹⁰)₂, steht,
- R⁹ für: 1. R⁸,
2. -(C₁-C₄)-Alkyl, worin Alkyl gerade oder verzweigt ist und unab-hängig voneinander ein-, zwei- oder dreifach substituiert ist durch
   2.1 Aryl, worin Aryl für einen Rest aus der Gruppe Phenyl, Naphthyl, Biphenylyl, Anthryl oder Fluorenyl steht und der Arylrest unsubstituiert ist oder einfach, zweifach oder dreifach, durch gleiche oder verschiedene Reste aus der Reihe -(C₁-C₈)-Alkyl, -(C₁-C₈)-Alkoxy, Halogen, Nitro, Amino, Trifluormethyl, Hydroxy, Hydroxy-(C₁-C₄)-alkyl wie Hydroxymethyl oder 1-Hydroxyethyl oder 2-Hydroxyethyl, Methylendioxy, Ethylendioxy, Formyl, Acetyl, Cyano, Hydroxycarbonyl, Aminocarbonyl, -(C₁-C₄)-Alkoxycarbonyl, Phenyl, Phenoxy, Benzyl, Benzyloxy oder Tetrazolyl substituiert ist,
   2.2 Halogen,
   2.3 -CN oder
   2.4 -CF₃,
3. Aryl, worin Aryl wie oben definiert ist und unsubstituiert oder wie oben substituiert ist, steht,
- R¹⁰ für: a) Wasserstoffatom,
b) -(C₁-C₆)-Alkyl, worin Alkyl unsubstituiert oder ein- bis dreifach unabhängig voneinander substituiert ist durch
   1. Aryl, worin Aryl wie oben definiert ist,
   2. Heteroaryl mit 5 bis 14 Ringgliedem, worin Heteroaryl wie oben definiert ist,
   3. Heterocyclus mit 5 bis 12 Ringgliedem, worin Heterocyclus wie oben definiert ist,
   4. Halogen,
   5. -N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -C(O)-OH substituiert ist, oder
   6. -C(O)-OH,
c) Aryl, worin Aryl wie oben definiert ist,
d) Heteroaryl mit 5 bis 14 Ringgliedern, worin Heteroaryl wie oben definiert ist, oder
e) Heterocyclus mit 5 bis 12 Ringgliedem, worin Heterocyclus wie oben definiert ist, steht und
für den Fall des (R¹⁰)₂ hat R¹⁰ unabhängig voneinander die Bedeutung von a) bis e),
- Z für: 1. 1,3,4-Oxadiazol, worin 1,3,4-Oxadiazol unsubstituiert oder ein- bis dreifach substituiert ist durch -NH₂, OH oder -(C₁-C₄)-Alkyl oder
2. -C(O)-R¹¹ steht, worin
- R¹¹ für: 1. -O-R¹⁰ oder
2. -N(R¹⁰)₂ steht, oder
- R⁷ und R⁸: bilden zusammen mit dem Stickstoff- und Kohlenstoffatom an das sie jeweils gebunden sind einen Ring der Formel IIa 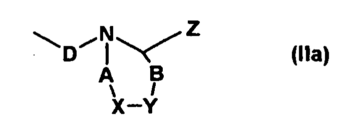 aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, Oxazol, Tetrazol, 1,2,3,5-Oxathiadiazol-2-Oxide, Triazolone, Oxadiazolone, Isoxazolone, Oxadiazolidindione, Triazole, welche unsubstituiert oder durch F, -CN, -CF₃ oder C(O)-O-(C₁-C₄)-Alkyl substituert sind, 3-Hydroxypyrro-2,4-dione, 5-Oxo-1,2,4-Thiadiazole, Isoxazol, 2-Isoxazolidin, Isoxazolidin, Morpholin, Isothiazol, Thiazol, Isothiazolidin, Thiomorpholin, Indazol, Thiadiazol, Benzimidazol, Chinolin, Triazol, Phthalazin, Chinazolin, Chinoxalin, Purin, Pteridin, Indol. Isochinolin, Tetrahydrochinolin und Tetrahydroisochinolin, oder
- R⁸ und Z: bilden zusammen mit den Kohlenstoffatomen an das sie jeweils gebunden sind einen Ring der Formel IIc 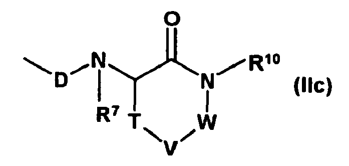 aus der Gruppe Pyrrol, Pyrrolin, Pyrrolidin, Pyridin, Piperidin, Pyrazolin, Phthalazin, Piperylen, Pyridazin, Pyrimidin, Pyrazin, Piperazin, Pyrazol, Imidazol, 1,3,4-Oxadiazol, Imidazolin, Pyrazolidin, Imidazolidin, Oxazol, Isoxazol, 2-Isoxazolidin, Isoxazolidin, Morpholin, Isothiazol, Thiazol, Isothiazolidin, Thiomorpholin, Indazol, Thiadiazol, Benzimidazol, Chinolin, Triazol, Tetrazol, 1,2,3,5-Oxathiadiazol-2-Oxide, Oxadiazolone, Isoxazolone, Triazolone, Oxadiazolidindione, Triazole, welche unsubstituiert oder durch F, -CN, -CF₃ oder -C(O)-O-(C₁-C₄)-Alkyl substituiert sind, 3-Hydroxypyrro-2,4-dione, 5-Oxo-1,2,4-Thiadiazole, Chinazolin, Chinoxalin, Purin, Indol, Pteridin, Tetrahydrochinolin, Tetrahydroisochinolin und Isochinolin, und
- R¹ R² und R⁴: für Wasserstoffatom stehen,
- R⁵ für: Wasserstoffatom steht und
- R⁶ für: 1. Phenyl, ein- oder zweifach unabhängig voneinander substituiert durch
   1.1 -CN,
   1.2 -CF₃,
   1.3 Halogen,
   1.4 -O-R¹⁰,
   1.5 -N(R¹⁰)₂,
   1.6 -NH-C(O)-R¹¹,
   1.7 -S(O)ₓ -R¹⁰, worin x die ganze Zahl Null, 1 oder 2 ist,
   1.8 -C(O)-R¹¹ oder
   1.9 -(C₁-C₄)-Alkyl-NH₂,
2. Heteroaryl mit 5 bis 14 Ringgliedern, worin Heteroaryl wie oben definiert ist und unsubstituiert oder ein-, zwei- oder dreifach unabhängig voneinander substituiert ist durch die oben unter 1.1 bis 1.9 definierten Substituenten oder
3. Heterocyclus mit 5 bis 12 Ringgliedem, worin Heterocyclus wie oben definiert ist und unsubstituiert oder ein-, zwei- oder dreifach unabhängig voneinander substituiert ist durch die oben unter 1.1 bis 1.9 definierten Substituenten, steht.

Insbesondere bevorzugt ist eine Verbindung der Formel I, wobei dass R³ für einen Rest der Formel II steht, worin
- D für: -C(O)- steht,
- R⁷ für: Wasserstoffatom steht,
- Z für: -C(O)-OH oder -C(O)-NH₂ steht,
- R⁸ für: 1. -(C₁-C₄)-Alkyl, worin Alkyl gerade oder verzweigt ist und ein- oder zweifach unabhängig voneinander substituiert ist durch
   1.1 -S(O)-R¹⁰, wobei R¹⁰ wie unten definiert ist,
   1.2 -N(R¹⁰)₂, wobei R¹⁰ wie unten definiert ist, oder
   1.3 Pyrrol, oder
2. für den charakteristischen Rest eine Aminosäure aus der Gruppe Histidin, Tryptophan, Serin, Threonin, Cystein, Methionin, Asparagin, Glutamin, Lysin, Arginin, Glutaminsäure und Asparaginsäure, steht
- R⁹ für: 1. Wasserstoffatom,
2. -(C₁-C₄)-Alkyl, worin Alkyl gerade oder verzweigt ist und unabhängig voneinander ein-, zwei- oder dreifach substituiert ist durch -C(O)-OH, -OH oder -C(O)-NH₂, oder
3. Phenyl, worin Phenyl unsubstituiert oder ein bis dreifach unabhängig voneinander substituiert ist durch Halogen oder -(C₁-C₄)-Alkyl, steht
- R¹⁰ für: a) Wasserstoffatom,
b) -(C₁-C₆)-Alkyl, worin Alkyl unsubstituiert oder ein bis dreifach unabhängig voneinander substituiert ist durch Halogen,
c) Phenyl, worin Phenyl unsubstituiert oder ein bis dreifach unabhängig voneinander substituiert ist durch Halogen oder -(C₁-C₄)-Alkyl,
- R¹, R² und R⁴: für Wasserstoffatom stehen,
- R⁵ für: Wasserstoffatom steht, und
- R⁶ für: Phenyl oder Pyridin steht.

Unter dem Begriff "Halogen" wird Fluor, Chlor, Brom oder Jod verstanden. Unter den Begriffen "-(C₁-C₈)-Alkyl", "-(C₁-C₆)-Alkyl" oder "-(C₁-C₄)-Alkyl" werden Kohlenwasserstoffreste verstanden, deren Kohlenstoffkette geradkettig oder verzweigt ist und 1 bis 8, 1 bis 6 bzw 1 bis 4 Kohlenstoffatome enthält. Cyclische Alkylreste sind beispielsweise 3- bis 6-gliedrige Monocyclen wie Cyclopropyl, Cyclobutyl, Cyclopentyl oder Cyclohexyl.

Unter dem Begriff "R⁷ und R⁸ bilden zusammen mit dem Stickstoff- und Kohlenstoffatom an das sie jeweils gebunden sind einen heterocyclischen Ring der Formel IIa" werden Reste verstanden die sich von Pyrrol, Pyrrolin, Pyrrolidin, Imidazol, Pyrazol, Oxazol, Isoxazol, Tetrazol, Isoxazolin, Isoxazolidin, Morpholin, Thiazol, Isothiazol, Isothiazolin, Purin, Isothiazolidin, Thiomorpholin, Pyridin, Piperidin, Pyrazin, Piperazin, Pyrimidin, Pyridazin, Indol, Isoindol, Indazol, Benzimidazol, Phthalazin, Chinolin, Isochinolin, Chinoxalin, Chinazolin, Cinnolin, Pteridin, Triazolone, Tetrazol, 1,2,3,5-Oxathiadiazol-2-Oxide, Oxadiazolone, Isoxazolone, Oxadiazolidindione, Triazole, welche durch F, -CN, -CF₃ oder-C(O)-O-(C₁-C₄)-Alkyl substituiert sind, 3-Hydroxypyrro-2,4-dione, 5-Oxo-1,2,4-Thiadiazole, Imidazolidin, -Carbolin und benz-anellierte Derivate dieser Heterocyclen ableiten.

Unter dem Begriff Aryl werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste. Arylreste, insbesondere Phenylreste, können einfach oder mehrfach, bevorzugt einfach, zweifach oder dreifach, durch gleiche oder verschiedene Reste substituiert sein, bevorzugt durch Reste aus der Reihe -(C₁-C₈)-Alkyl, insbesondere -(C₁-C₄)-Alkyl, -(C₁-C₈)-Alkoxy, insbesondere -(C₁-C₄)-Alkoxy, Halogen, Nitro, Amino, Trifluormethyl, Hydroxy, Hydroxy-(C₁-C₄)-alkyl wie Hydroxymethyl oder 1-Hydroxyethyl oder 2-Hydroxyethyl, Methylendioxy, Ethylendioxy, Formyl, Acetyl, Cyano, Hydroxycarbonyl, Aminocarbonyl, -(C₁-C₄)-Alkoxycarbonyl, Phenyl, Phenoxy, Benzyl, Benzyloxy, Tetrazolyl substituiert sein. Entsprechendes gilt beispielsweise für Reste wie Arylalkyl oder Arylcarbonyl. Arylalkylreste sind insbesondere Benzyl sowie 1- und 2-Naphthylmethyl, 2-, 3- und 4-Biphenylylmethyl und 9-Fluorenylmethyl. Substituierte Arylalkylreste sind beispielsweise durch einen oder mehrere -(C₁-C₈)-Alkylreste, insbesondere -(C₁-C₄)-Alkylreste, im Arylteil substituierte Benzylreste und Naphthylmethylreste, zum Beispiel 2-, 3- und 4-Methylbenzyl, 4-Isobutylbenzyl, 4-tert-Butylbenzyl, 4-Octylbenzyl, 3,5-Dimethylbenzyl, Pentamethyl-benzyl, 2-, 3-, 4-, 5-, 6-, 7- und 8-Methyl-1-naphthylmethyl, 1-, 3-, 4-, 5-, 6-, 7- und 8-Methyl-2-naphthylmethyl, durch einen oder mehrere -(C₁-C₈)-Alkoxyreste, insbesondere -(C₁-C₄)-Alkoxyreste, im Arylteil substituierte Benzylreste und Naphthylmethylreste, zum Beispiel 4-Methoxybenzyl, 4-Neopentyloxybenzyl, 3,5-Dimethoxybenzyl, 3,4-Methylen-dioxybenzyl, 2,3,4-Trimethoxybenzyl, Nitrobenzylreste, zum Beispiel 2-, 3- und 4-Nitrobenzyl, Halobenzylreste, zum Beispiel 2-, 3- und 4-Chlor- und 2-, 3-, und 4-Fluorbenzyl, 3,4-Dichlorbenzyl, Pentafluorbenzyl, Trifluormethylbenzylreste, zum Beispiel 3- und 4-Trifluormethylbenzyl oder 3,5-Bis(trifluormethyl)benzyl.

In monosubstituierten Phenylresten kann sich der Substituent in der 2-Position, der 3-Position oder der 4-Position befinden. Zweifach substituiertes Phenyl kann in der 2,3-Position, der 2,4-Position, der 2,5-Position, der 2,6-Position, der 3,4-Position oder der 3,5-Position substituiert sein. In dreifach substituierten Phenylresten können sich die Substituenten in der 2,3,4-Position, der 2,3,5-Position, der 2,4,5-Position, der 2,4,6-Position, der 2,3,6-Position oder der 3,4,5-Position befinden.

Die Erläuterungen zu den Arylresten gelten entsprechend für zweiwertige Arylenreste, zum Beispiel für Phenylenreste, die beispielsweise als 1,4-Phenylen oder als 1,3-Phenylen vorliegen können.
Phenylen-(C₁-C₆)-alkyl ist insbesondere Phenylenmethyl (-C₆H₄-CH₂-) und Phenylenethyl, (C₁-C₆)-Alkylen-phenyl insbesondere Methylenphenyl (-CH₂-C₆H₄-). Phenylen-(C₂-C₆)-alkenyl ist insbesondere Phenylenethenyl und Phenylenpropenyl.

Der Begriff "Heteroaryl mit 5 bis 14 Ringgliedern" steht für einen Rest eines monocyclischen oder polycyclischen aromatischen Systems mit 5 bis 14 Ringgliedern, das 1, 2, 3, 4 oder 5 Heteroatome als Ringglieder enthält. Beispiele für Heteroatome sind N, O und S. Sind mehrere Heteroatome enthalten, können diese gleich oder verschieden sein. Heteroarylreste können ebenfalls einfach oder mehrfach, bevorzugt einfach, zweifach oder dreifach, durch gleiche oder verschiedene Reste aus der Reihe -- -(C₁-C₈)-Alkyl, insbesondere -(C₁-C₄)-Alkyl, -(C₁-C₈)-Alkoxy, insbesondere -(C₁-C₄)-Alkoxy, Halogen, Nitro, -N(R¹⁰)₂, Trifluormethyl, Hydroxy, Hydroxy-(C₁-C₄)-alkyl wie Hydroxymethyl oder 1-Hydroxyethyl oder 2-Hydroxyethyl, Methylendioxy, Formyl, Acetyl, Cyano, Hydroxycarbonyl, Aminocarbonyl, -(C₁-C₄)-Alkoxycarbonyl, Phenyl, Phenoxy, Benzyl, Benzyloxy, Tetrazolyl substituiert sein. Bevorzugt steht Heteroaryl mit 5 bis 14 Ringgliedern für einen monocyclischen oder bicyclischen aromatischen Rest, der 1, 2, 3 oder 4, insbesondere 1, 2 oder 3, gleiche oder verschiedene Heteroatome aus der Reihe N, O und S enthält und der durch 1, 2, 3 oder 4, insbesondere 1 bis 3, gleiche oder verschiedene Substituenten aus der Reihe -(C₁-C₆)-Alkyl, -(C₁-C₆)-Alkoxy, Fluor, Chlor, Nitro, -N(R¹⁰)₂, Trifluormethyl, Hydroxy, Hydroxy-(C₁-C₄)-alkyl, -(C₁-C₄)-Alkoxycarbonyl, Phenyl, Phenoxy, Benzyloxy und Benzyl substituiert sein kann. Besonders bevorzugt steht Heteroaryl für einen monocyclischen oder bicyclischen aromatischen Rest mit 5 bis 10 Ringgliedern, insbesondere für einen 5-gliedrigen bis 6-gliedrigen monocyclischen aromatischen Rest, der 1, 2 oder 3, insbesondere 1 oder 2, gleiche oder verschiedene Heteroatome aus der Reihe N, O und S enthält und durch 1 oder 2 gleiche oder verschiedene Substituenten aus der Reihe -(C₁-C₄)-Alkyl, Halogen, Hydroxy, -N(R¹⁰)₂, -(C₁-C₄)-Alkoxy, Phenyl, Phenoxy, Benzyloxy und Benzyl substituiert sein kann.

Der Begriff "Heterocyclus mit 5 bis 12 Ringgliedern" steht für einen monocyclischen oder bicyclischen 5-gliedrigen bis 12-gliedrigen heterocyclischen Ring, der teilweise gesättigt oder vollständig gesättigt ist. Beispiele für Heteroatome sind N, O und S. Der Herterocyclus ist unsubstituiert oder an einem oder mehreren Kohlenstoffatomen oder an einem oder mehreren Heteroatomen durch gleiche oder verschiedene Substituenten substituiert. Diese Substituenten sind oben beim Rest Heteroaryl definiert worden. Insbesondere ist der heterocyclische Ring einfach oder mehrfach, zum Beispiel einfach, zweifach, dreifach oder vierfach, an Kohlenstoffatomen durch gleiche oder verschiedene Reste aus der Reihe -(C₁-C₈)-Alkyl, zum Beispiel -(C₁-C₄)-Alkyl, -(C₁-C₈)-Alkoxy, zum Beispiel -(C₁-C₄)-Alkoxy wie Methoxy, Phenyl-(C₁-C₄)-alkoxy, zum Beispiel Benzyloxy, Hydroxy, Oxo, Halogen, Nitro, Amino oder Trifluormethyl substituiert und/oder er ist an den Ring-Stickstoffatome/en im heterocyclischen Ring durch -(C₁-C₈)-Alkyl, zum Beispiel -(C₁-C₄)-Alkyl wie Methyl oder Ethyl, durch gegebenenfalls substituiertes Phenyl oder Phenyl-(C₁-C₄)-alkyl, zum Beispiel Benzyl, substituiert. Stickstoffheterocyclen können auch als N-Oxide vorliegen oder als Quartärsalze.

Beispiele für die Begriffe Heteroaryl mit 5 bis 14 Ringgliedern oder Heterocyclus mit 5 bis 12 Ringgliedern sind Reste, die sich von Pyrrol, Furan, Thiophen, Imidazol, Pyrazol, Oxazol, Isoxazol, Thiazol, Isothiazol, Tetrazol, 1,2,3,5-Oxathiadiazol-2-Oxide, Triazolone, Oxadiazolone, Isoxazolone, Oxadiazolidindione, Triazole, weiche durch F, - CN, -CF₃ oder-C(O)-O-(C₁-C₄)-Alkyl substituert sind, 3-Hydroxypyrro-2,4-dione, 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, -Carbolin und benz-anellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Die allgemeine Strukturformel von α-Aminosäuren ist wie folgt:

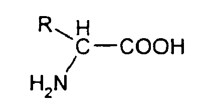

Die α-Aminosäuren unterscheiden sich untereinander durch den Rest R, der im Rahmen der vorliegenden Anmeldung als "charakteristischer Rest " einer Aminosäure bezeichnet wird.
Für den Fall, daß R⁸ den charakteristischen Rest einer Aminosäure bedeutet, werden vorzugsweise die charakteristischen Reste der folgenden natürlich vorkommenden α-Aminosäuren Glycin, Alanin, Valin, Leucin, Isoleucin, Phenylalanin, Tyrosin, Tryptophan, Serin, Threonin, Cystein, Methionin, Asparagin, Glutamin, Lysin, Histidin, Arginin, Glutaminsäure und Asparaginsäure eingesetzt. Insbesondere bevorzugt sind Histidin, Tryptophan, Serin, Threonin, Cystein, Methionin, Asparagin, Glutamin, Lysin, Arginin, Glutaminsäure und Asparaginsäure. Ferner sind bevorzugte charakteristische Reste einer Aminosäure die eingesetzt werden als Rest R⁸ auch nicht natürlich vorkommenden Aminosäuren wie 2-Aminoadipinsäure, 2-Aminobuttersäure, 2-Aminoisobuttersäure , 2,3-Diamino-propionsäure, 2,4-Diaminobuttersäure, 1,2,3,4-Tetrahydroisochinolin-1-carbonsäure, 1,2,3,4-Tetrahydroisochinolin-3-carbonsäure, 2-Aminopimelinsäure, Phenylglycin, 3-(2-Thienyl)-alanin, 3-(3-Thienyl)-alanin, 2-(2-Thienyl)-glycin, 2-Amino-heptansäure, Pipecolinsäure, Hydroxylysin, Sarkosin, N-Methylisoleucin, 6-N-Methyl-lysin, N-Methylvalin, Norvalin, Norleucin, Ornithin, allo-Isoleucin, allo-Threonin, allo-Hydroxylysin, 4-Hydroxyprolin, 3-Hydroxyprolin, 3-(2-Naphtyl)-alanin, 3-(1-Naphtyl-alanin), Homophenylalanin, Homocystein, Homocysteinsäure, Homotryptophan, Cysteinsäure, 3-(2-Pyridyl)-alanin, 3-(3-Pyridyl)-alanin, 3-(4-Pyridyl)-alanin, 2-Amino-3-phenylaminopropionsäure, 2-Amino-3-phenylaminoethylpropionsäure, Phosphinothricin, 4-Fluorphenylalanin, 3-Fluorphenylalanin, 4-Fluorphenylalanin, 3-Fluorphenylalanin, 3-Fluorphenylalanin, 2-Fluor-phenylalanin, 4-Chlorphenylalanin, 4-Nitrophenylalanin, 4-Aminophenylalanin, Cyclohexylalanin, Citrullin, 5-Fluortryptophan, 5-Methoxytryptophan, Methionin-Sulfon, Methionin-Sulfoxid oder -NH-NR¹⁰-C(O)N(R¹⁰)₂, die gegebenenfalls auch substituiert sind. Bei natürlichen aber auch nicht natürlichen vorkommenden Aminosäuren, die eine funktionelle Gruppe wie Amino, Hydroxy, Carboxy, Mercapto, Guanidyl, Imidazolyl oder Indolyl haben, kann diese Gruppe auch geschützt sein.

Als geeignete Schutzgruppe werden dafür vorzugsweise die in der Peptidchemie gebräuchlichen N-Schutzgruppen verwendet, beispielsweise Schutzgruppen vom Urethan-Typ, Benzyloxycarbonyl (Z), t-Butyloxycarbonyl (Boc), 9-Fluorenyloxycarbonyl (Fmoc), Allyloxycarbonyl (Aloc) oder vom Säureamid-Typ insbesondere Formyl, Acetyl oder Trifluoracetyl sowie vom Alkyl-Typ beispielsweise Benzyl. Für den Fall eines Imidazols-Restes in R⁸ dient beispielsweise das für die Sulfonamidbildung eingesetzte Sulfonsäurederivat der Formel IV als Schutzgruppe des Imidazol-Stickstoffs, die sich insbesondere in Gegenwart von Basen wie Natronlauge wieder abspalten läßt.
Die Ausgangsstoffe der chemischen Umsetzungen sind bekannt oder lassen sich nach literaturbekannten Methoden leicht herstellen.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, daß man
a) eine Verbindung der Formel IV, 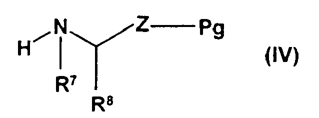 worin Pg ein geeignete Schutzgruppe (z.B. Methylester), eine Amidgruppe oder eine Hydroxy-Gruppe darstellt und Z, R⁷ und R⁸ wie in Formel I definiert sind, mit einem Säurechlorid oder einem aktivierten Ester der Verbindung der Formel III, 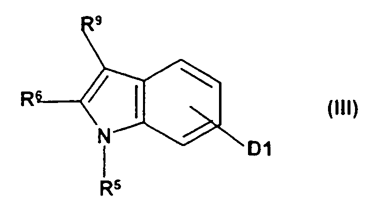 wobei D1 -COOH oder Sulfonylhalogen bedeutet und R⁵ , R⁶ und R⁹ wie in Formel I definiert sind, in Gegenwart einer Base oder gegebenenfalls eines wasserentziehenden Mittels in Lösung umsetzt und nach Abspaltung der Schutzgruppe in eine Verbindung der Formel I überführt, oder
b) eine Verbindung der Formel IVa, 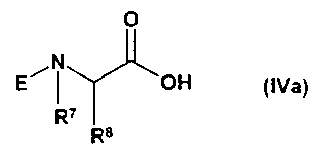 worin R⁷ und R⁸ wie in Formel I definiert sind und E eine N-Aminoschutzgruppe darstellt, mit ihrer Carboxylgruppe über eine Zwischenkette L an ein polymeres Harz der allgemeinen Formel PS ankoppelt, wobei eine Verbindung der Formel V 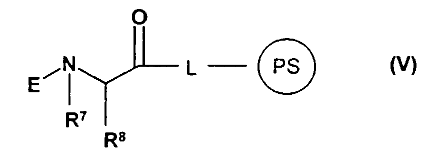 entsteht, die nach selektiver Abspaltung der Schutzgruppe E mit einer Verbindung der Formel III, wobei R⁵, R⁶ und R⁹ wie in Formel I definiert sind, in Gegenwart einer Base oder gegebenenfalls eines wasserentziehenden Mittels zu einer Verbindung der Formel VI 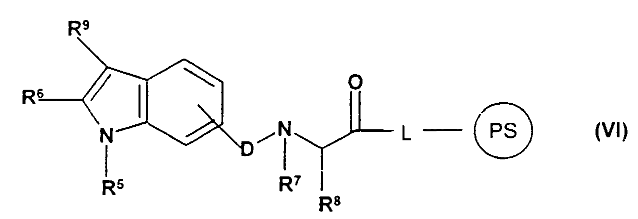 umsetzt, und die Verbindung der Formel VI nach Abspaltung vom Trägermaterial in eine Verbindung der Formel I überführt, oder
c) eine Verbindung der Formel I in ein physiologisch verträgliches Salz überführt.

Nach der Verfahrensvariante a) werden die Säurefunktionen der Verbindungen der Formel IVa mit einer Schutzgruppe Pg versehen, diese selektive Carbonsäuren-Derivatisierung erfolgt nach Methoden wie sie in Houben-Weyl "Methoden der Org. Chemie", Band 15/1 beschrieben sind. Inder Verfahrensvariante b) wird die Aminofunktionen der Ausgangsverbindungen der Formeln mit einer Schutzgruppe E versehen, diese selektive Aminogruppen-Derivatisierung erfolgt nach Methoden wie sie in Houben-Weyl "Methoden der Org. Chemie", Band 15/1 beschrieben sind.
Als geeignete Schutzgruppe Pg wird dafür vorzugsweise die in der Peptidchemie gebräuchlichen Carboxy-Schutzgruppen verwendet, beispielsweise Schutzgruppen vom Alkylester-Typ, wie Methyl-, Ethyl-, tert.-Butyl-, iso-Propyl-, Benzyl-, Fluorenylmethyl-, Allyl-, Arylester-Typ, wie Phenyl-, Amid-Typ, wie Amid- oder Benzhydrylamin. Als geeignete Schutzgruppe E wird dafür vorzugsweise die in der Peptidchemie gebräuchlichen N-Schutzgruppen verwendet, beispielsweise Schutzgruppen vom Urethan-Typ, wie Benzyloxycarbonyl(Z), t-Butyloxycarbonyl (Boc), 9-Fluorenylmethoxycarbonyl (Fmoc) und Allyloxycarbonyl (Aloc) oder von Säureamid-Typ insbesondere Formyl, Acetyl oder Trifluoracetyl von Alkyl-Typ wie Benzyl.
Besonders geeignet hat sich dafür auch die (Trimethyl-silyl)ethoxycarbonyl (Teoc)Gruppe (P. Kociénski, Protecting Groups, Thieme Verlag 1994).

Die Darstellung der Indolcarbonsäure-Derivate erfolgt nach einer Methode wie sie in Houben-Weyl "Methoden der Org. Chemie", Band E6-2A bzw. E6-2B beschrieben ist. So lassen sich zur Darstellung der Indolcarbonsäure-Derivate der Formel III bevorzugt Hydrazinobenzoesäuren und Aryl- oder Heteroarylketone, in Gegenwart von Polyphosphorsäure als Lösungsmittel bei 145 °C umsetzen. Die Darstellung der benötigten Hydrazinobenzoesäuren erfolgt nach dem Fachmann geläufigen Methoden z.B. aus den entsprechenden Benzoesäure-anilinen, Aryl- oder Heteroarylketone werden ebenfalls nach dem Fachmann gängigen Methoden z.B. aus den entsprechenden Säurechloriden oder Nitrilen durch Umsetzung mit z.B. Organometall-Verbindungen hergestellt.

Zur Kondensation der Verbindungen der Formel IV mit denen der Formel III verwendet man vorteilhafterweise die dem Fachmann an sich wohlbekannten Kupplungsmethoden der Peptidchemie (siehe z.B Houben-Weyl, Methoden der Organischen Chemie, Band 15/1 und 15/2, Georg Thieme Verlag, Stuttgart, 1974). Als Kondensationsmittel oder Kupplungsreagenzien kommen Verbindungen wie Carbonyldiimidazol, Carbodiimide wie Dicyclohexylcarbodiimid oder Diisopropylcarbodiimid (DIC), das O-((Cyano(ethoxycarbonyl)-methylen)amino)-N,N,N',N'-tetramethyluronium-tetrafluoroborat (TOTU) oder Propylphosphonsäureanhydrid (PPA) in Frage.

Die Kondensationen können unter Standardbedingungen durchgeführt werden. Bei der Kondensation ist es in der Regel nötig, daß die vorhandenen, nicht reagierenden Aminogruppen durch reversible Schutzgruppen geschützt werden. Gleiches gilt für nicht an der Reaktion beteiligte Carboxylgruppen, die während der Kondensation bevorzugt als -(C₁-C₆)-Alkylester, Benzylester oder tert-Butylester vorliegen. Ein Aminogruppen-Schutz erübrigt sich, wenn die Aminogruppen noch in Form von Vorstufen wie Nitrogruppen oder Cyanogruppen vorliegen und erst nach der Kondensation durch Hydrierung gebildet werden. Nach der Kondensation werden die vorhandenen Schutzgruppen in geeigneter Weise abgespalten. Beispielsweise können NO₂-Gruppen (Guanidinoschutz in Aminosäuren), Benzyloxycarbonylgruppen und Benzylgruppen in Benzylestern abhydriert werden. Die Schutzgruppen vom tert-Butyltyp werden sauer abgespalten, während der 9-Fluorenylmethyloxycarbonylrest durch sekundäre Amine entfernt wird.

Der in den Formeln V und VI mit PS bezeichnete polymere Träger ist ein quervernetztes Polystyrolharz mit einem als Zwischenkette L bezeichneten Linker. Dieser Linker trägt eine geeignete funktionelle Gruppe, beispielsweise Amin bekannt beispielsweise als Rink-Amid Harz, oder eine OH-Gruppe, bekannt beispielsweise als Wang-Harz oder Kaiser's Oxim-Harz. Alternativ können andere polymere Träger wie Glas, Baumwolle oder Cellulose mit verschiedenen Zwischenketten L eingesetzt werden.
Die mit L bezeichnete Zwischenkette ist kovalent an den polymeren Träger gebunden und erlaubt eine reversible, amidartige oder esterartige Bindung mit der Verbindung der Formel IVa, die während der weiteren Umsetzung an der gebundenen Verbindung der Formel IVa stabil bleibt; jedoch unter stark sauren Reaktionsbedingungen, z.B. Mischungen mit Trifluoressigsäure, die am Linker befindliche Gruppe wieder freisetzt.

Die Freisetzung der gewünschten Verbindung der allgemeinen Formel I vom Linker kann an verschiedenen Stellen in der Reaktionsfolge geschehen.

### A. Allgemeine Vorgehensweise zur Kopplung von geschützten Aminocarbonsäuren der Formel IVa an den festen Träger :

Die Synthese wurde in Reaktoren mit je 15 ml Reaktionsvolumen durchgeführt.
Jeder der Reaktoren wurde mit 0,179 g Rink-Amid-AM Harz (Fmoc-Rink-Amid AM/ Nova-Biochem; Beladung 0,56 mmol/g; d.h. 0,1 mmol/Reaktor) befüllt. Zur Abspaltung der Fmoc-Schutzgruppe vom Harz wurden in jeden Reaktor eine 30%ige Piperidin / DMF-Lösung zudosiert und die Mischung 45 Minuten (Min) lang geschüttelt. Anschließend wurde filtriert und das Harz mit Dimethylformamid (DMF) 3 mal gewaschen.
Zur Kopplung der geschützten Aminosäure wurden zu dem so vorbereiteten Harz je eine 0,5 molare Lösung der entsprechenden Fmoc-Aminosäure (0,3 mmol in DMF); Lösung von HOBt (0,33 mmol in DMF) und eine Lösung von DIC (0,33 mmol in DMF) zudosiert und die Mischung 16 Stunden (h) bei 35°C geschüttelt. Anschließend wurde das Harz mehrmals mit DMF gewaschen.
Zur Überprüfung der Kopplung wurden einige Harzkügelchen entnommen und einem KAISER-Test unterworfen; in allen Fällen war der Test negativ.
Die Abspaltung der Fmoc-Schutzgruppe erfolgte, wie oben erwähnt, mit 30% iger Piperidin/DMF-Lösung.
Zur Kopplung der Indol-carbonsäuren wurde eine 0,1 molare Lösung der entsprechenden 4- oder 5- substituierten Säure (0,4 mmol in DMF); eine 0,5 molare Lösung des Kopplungsreagenzes TOTU (0,44 mmol in DMF) und eine 0,5 molare Lösung DIPEA (0,6 mmol in DMF) zudosiert und die Mischung 16 Stunden bei 40°C geschüttelt. Anschließend wurde mehrmals mit DMF gewaschen.
Zur Reaktionskontrolle wurden wiederum einige Harzkügelchen entnommen und einem KAISER-Test unterworfen.
Zur Abspaltung der gewünschten Substanzen vom festen Träger wurde das Harz mehrmals mit Dichlormethan gewaschen. Anschließend wurde die Abspaltlösung (50% Dichlormethan und 50% einer Mischung aus 95 % TFA, 2 % H₂O, 3 % Triisopropylsilan) zudosiert und die Mischung 1 h bei Raumtemperatur geschüttelt. Die Mischung wurde filtriert und das Filtrat bis zur Trockne eingeengt. Der Rückstand wurde mit Diethylether ausgefällt und fitriert.

Die festen Rückstände enthielten die gewünschten Produkte meist in hoher Reinheit oder wurden beispielsweise mit präparativer Hochdruck-Flüssigkeits-Chromatographie an einer reversen Phase (Eluentien: A: H₂O/ 0, 1 % TFA, B: Acetonitril/ 0.1% TFA) fraktioniert. Lyophilisation der erhaltenen Fraktionen lieferte die gewünschten Produkte.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindungen der Formeln I basische Gruppen aufweist, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von I_{K}B-Kinase beteiligt ist. Dazu gehören beispielsweise chronische Erkrankungen des Bewegungsapparates wie entzündliche, immunologisch oder stoffwechselbedingte akute und chronische Arthritiden, Arthropathien, rheumatoide Arthritis, oder degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen oder Erkrankungen des Bindegewebes wie Kollagenosen und Periodontalerkrankungen, Myalgien und Störungen des Knochenstoffwechsels, oder Erkrankungen, die durch eine Überexpression von Tumor Nekrose Faktor alpha (TNFα) oder erhöhte Konzentration an TNFα bedingt sind wie Kachexie, Mutiple Sklerose, Schädel-Hirn Trauma, Morbus Crohn und Darmgeschwüre, oder Erkrankungen wie Atherosklerose, Stenosen, Ulceration, Alzheimers Erkrankungen, Muskelabbau, Krebserkrankungen (Potenzierung von Cytotoxica-Therapien), Herzinfarkt, Gicht, Sepsis, septischer Schock, endotoxischer Schock, virale Infektionen wie Grippe, Hepatitis, HIV-Infektionen, AIDS, oder durch Adenoviren oder Herpesviren verursachte Erkrankungen, parasitische Infektionen wie Malaria oder Lepra, Pilz- oder Hefeinfektionen, Gehirnhautentzündungen, chronische entzündliche Lungenerkrankungen wie chronische Bronchitis oder Asthma, acute respiratory distress syndrome, akute Synovitis, Tuberkulose, Psoriasis, Diabetes, Behandlung von akuten oder chronischen Abstoßungsreaktionen des Organempfängers gegen das verpflanzte Organ, chronische Graft-versus-Host-Erkrankungen und entzündliche Gefäßerkrankungen.

Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet, daß man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt. Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungs-einheiten wie Tabletten, Kapseln, Dragees oder Suppositorien, kann diese Dosis bis zu etwa 1000 mg, bevorzugt von etwa 50 mg bis 300 mg und bei Injektionslösungen in Ampullenform bis zu etwa 300 mg, vorzugsweise von etwa 10 mg bis 100 mg, betragen. Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt von etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Endprodukte werden in der Regel durch massenspektroskopische Methoden (FAB-, ESI-MS) bestimmt. Temperaturangaben in Grad Celsius, RT bedeutet Raumtemperatur (22 °C bis 26 °C). Verwendete Abkürzungen sind entweder erläutert oder entsprechen den üblichen Konventionen.

### Beispiele

### Darstellung substituierter Indolcarbonsäuren

### Verfahrensvariante A) 2,3-Diphenyl-1 H-indol-5-carbonsäure:

Es wurden 1,96 g (10 mMol) Deoxybenzoin und 1,52 g 4-Hydrazinobenzoesäure im Mörser kleingerieben und anschließend im offenen Kolben 15 Minuten (min) bei 160 °C zusammengeschmolzen. Die abgekühlte Schmelze wurde mit 100 ml Essigsäure und 30 ml konzentrierter Salzsäure versetzt und 3 Stunden (h) unter Rückfluß erhitzt. Beim Versetzen der abgekühlten Lösung mit Wasser fiel das Produkt 2,3-Diphenyl-1 H-indol-5-carbonsäure aus. Man saugte ab, wusch den Rückstand mit Wasser nach und trocknete. Zur Reinigung wurde das Rohprodukt mit warmem Toluol verrührt, abgesaugt und erneut getrocknet. Man erhielt 2,3-Diphenyl-1 H-indol-5-carbonsäure.

### Verfahrensvariante B)

### 2-Pyridin-4-yl-1 H-indol-5-carbonsäure:

Es wurden 20 g P₂O₅ mit 12,5 ml H₃PO₄ (85%) versetzt, wobei sich die Reaktionsmischung stark erwärmt. Danach wurde die Reaktionsmischung auf 60 °C abgekühlt und es wurden 8,90 g (65,84 mmol) 4-Propionylpyridin und 4,20 g (27,60 mMol) 4-Hydrazinobenzoesäure dazugegeben. Anschließend wurde 45 min bei 145 °C gerührt. Die Reaktionsmischung wurde auf Wasser gegossen, worauf das gelb gefärbte Produkt 2-Pyridin-4-yl-1 H-indol-5-carbonsäure ausfiel. Dieser Niederschlag wurde abgesaugt und mit Wasser neutral gewaschen. Die so in quantitativer Ausbeute erhaltene 2-Pyridin-4-yl-1 H-indol-5-carbonsäure wurde ohne weitere Reinigung für die Kopplung mit Aminosäure-derivaten benutzt.
Kopplung von Aminosäure-derivaten mit substituierten Indolcarbonsäurederivaten Verfahrensvariante C)

### Beispiel 1

### 2,3-Diphenyl-1H-indol-5-carbonsäure (1-carbamoyl-3-phenyl-propyl)-amid:

Es wurden 0,16 g (0,5 mMol) 2,3-Diphenyl-1 H-indol-5-carbonsäure (siehe Verfahrensvariante A) bei RT in 10 ml trockenem Dimethylformamid (DMF) gelöst und nacheinander mit 0,11 g (0,5 mMol) L-Homophenylalaninamid Hydrochlorid, 0,16 g TOTU (O-[(Cyano(ethoxycarbonyl)methyliden)amino-1,1,3,3-tetramethyl] uronium-tetrafluoro-borat) und 0,14 ml (1 mMol) Diisopropylamin versetzt. Man rührte 6 h bei RT. Das Reaktionsgemisch wurde unter verminderten Druck eingeengt und der Rückstand in Essigsäureethylester gelöst. Die organische Phase wurde nacheinander mit Wasser, gesättigter Natriumcarbonatlösung, Wasser und gesättigter Kochsalzlösung gewaschen, über Magnesiumsulfat getrocknet, filtriert und unter verminderten Druck eingeengt. Man erhielt 2,3-Diphenyl-1H-indol-5-carbonsäure (1-carbamoyl-3-phenyl-propyl)-amid mit dem Schmelzpunkt 120 °C bis 125 °C.

### Beispiel 7:

### 3-Methyl-2-pyridin-4-yl-1 H-indole-5-carboxylic acid (1-carbamoyl-3-pyrrol-1-yl-propyl)-amid

Es wurden 0,13 g (0,5 mMol) 3-Methyl-2-pyridin-4-yl-1 H-indole-5-carboxylic acid (siehe Verfahrensvariante A) bei RT in 10 ml trockenem Dimethylformamid (DMF) gelöst und nacheinander mit 0,083 g (0,5 mMol) 4-(1-Pyrrolyl)-L-2-benzyloxycarbonylamino-buttersäureamid, 0,16 g (0,5 mMol) TOTU (O[(Cyano(ethoxycarbonyl)-methyliden)amino-1,1,3,3,-tetramethyl] uronium-tetrafluoro-borat) und 0,14 ml (1 mMol) Ethyl-diisopropylamin versetzt. Man rührte 6 h bei RT. Das Reaktionsgemisch wurde unter verminderten Druck eingeengt und der Rückstand in Essigsäureethylester gelöst. Die organische Phase wurde nacheinander mit Wasser, gesättigter Natriumcarbonatlösung, Wasser und gesättigter Kochsalzlösung gewaschen, über Magnesiumsulfat getrocknet, filtriert und unter verminderten Druck eingeengt. Die Reinigung erfolgte mittels präp. HPLC.

### a: 4-(1-Pyrrolyl)-L-2-benzyloxycarbonylamino-buttersäure

Zu einer mit Argon gespülten Lösung v. 1,25 g (5,0 mMol) N_{α}-Z-L-2,4-diaminobuttersäure in 60 ml Wasser wurden 0,66 g (5,0 mMol) 2,5-Dimethoxytetrahydrofuran gefolgt von 1,7 ml Eisessig gegeben und 12 h bei 20°C gerührt. Das Reaktionsgemisch wurde mehrmals mit Ethylacetat extrahiert, die org. Phasen vereinigt, mit Natriumsulfat getrocknet und das Filtrat unter verminderten Druck eingeengt. Das Rohprodukt wurde durch Flash-Chromatographie an Kieselgel (CH₂Cl₂ / CH₃OH / CH₃COOH : 100 / 5 /1) gereinigt. Nach Entfernung des Elutionsmittels wurden 1,3 g (87%) 4-(1-Pyrrolyl)-L-2-benzyloxycarbonylamino-buttersäure erhalten.

### b: 4-(1-Pyrrolyl)-L-2-benzyloxycarbonylamino-buttersäureamid

1,2 g (4,0 mMol) 4-(1-Pyrrolyl)-L-2-benzyloxycarbonylamino-buttersäure und 0,61 g (4,0 mMol) N-Hydroxybenzotriazol-ammoniumsalz wurden zusammen in 10 ml DMF gelöst, bei 0°C mit 0.82 g (4,0 mMol) N,N'-Dicyclohexylcarbodiimid und 0,68 ml (4,0 mMol) N-Ethyl-diisopropylamin versetzt , 30 min bei 0°C und 3 h bei 20°C gerührt. Der ausgefallene Harnstoff wurde abgesaugt und das Filtrat i.V. zur Trockene eingedampft. Das Rohprodukt wurde durch Chromatographie an Kieselgel (CH₂Cl₂ / CH₃OH / CH₃COOH : 100 / 5 /1) gereinigt. Ausbeute: 0,89 g (74%)

### c: 4-(1-Pyrrolyl)-L-2-amino-buttersäureamid

0,80 g (2,65 mMol) 4-(1-Pyrrolyl)-L-2-benzyloxycarbonylamino-buttersäureamid gelöst in 20 ml Methanol wurden unter Inertgas mit 80 mg Katalysator (10% Pd-C) versetzt, anschl. wurde bis zur vollständigen Abspaltung der Z-schutzgruppe Wasserstoff eingeleitet. Nach Abfiltrieren des Katalysators und eindampfen des Filtrates wurden 0,4 g (90,5%) 4-(1-Pyrrolyl)-L-2-amino-buttersäureamid erhalten.

### 2. Verfahrensvariante D)

### Beispiel 3:

### 2-Pyridin-4-yl-1H-indol-5-carbonsäure(1-carbamoyl-2-phenylsulfanyl-ethyl)-amid

Zu 0,20 g (0,84 mMol) 2-Pyridin-4-yl-1 H-indol-5-carbonsäure wurde 0,21 g (1,07 mMol) 2-Amino-3-phenylsulfanyl-propionsäure in 40 ml DMF zugegeben und bei 0 °C wurden 0,66 g (1,27 mMol) Benzotriazol-1-yloxy-tripyrrolidino-phosphonium-hexafluoro-phosphat und 0,37 ml (2,12 mMol) N-Ethyl-N,N-diisopropylamin zugegeben und man rührte 2 h bei 20 °C. Die Lösung wurde unter verminderten Druck eingeengt und mittels Mitteldruck Säulen Chromatographie (CH₂Cl₂ / CH₃OH wie 9 : 1) gereinigt. Es wurden so 0,19 g (54%) 2-Pyridin-4yl-1H-indol-5-carbonsäure(1-carbamoyl-2-phenylsulfanyl-ethyl)-amid erhalten.

### Beispiel 9 3-Phenylaminoethyl-2-[(2-pyridin-4-yl-1 H-indol-5-carbonyl)-amino]-propionsäureamid

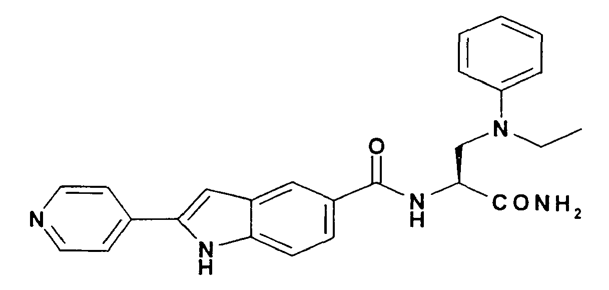

### a) L-2-Amino-3-phenylaminoethylpropionsäure

54,8 g (0,209 mol) Triphenylphosphin wurden in 600 ml Acetonitril suspendiert und unter Ausschluss von Feuchtigkeit auf -35 °C bis -45 °C gekühlt. Anschließend wurde bei dieser Temperatur innerhalb von 50 min tropfenweise 36,4 g (0,209 mol) Azodicarbonsäure-diethylester hinzugegeben. Man rührte 15 min bei -35°C nach. Zu diesem Gemisch tropfte man eine Lösung aus 50 g (0,209 mol) N-Benzyloxycarbonyl-L-serin in 500 ml Acetonitril, dabei ließ man die Temperatur nicht über -35°C steigen. Anschließend ließ man 12 h bei 5 °C nachreagieren und erwärmte auf RT. Die Reaktionslösung wurde unter vermindertem Druck vom Lösungsmittel befreit und das Rohprodukt mit Mitteldruckchromatographie an Kieselgel gereinigt. (DCM/AcCN : 25/1) Nach Entfernen des Lösungsmittels erhielt man 20,8 g (Ausbeute 45%) sauberes N-Benzyloxy-carbonyl-L-serin-ß-lacton (siehe auch Org. Synth. 1991 (70) 1ff.) in feinen Nadeln. Summenformel C₁₁H₁₁NO₄;M.W. = 221,2; MS (M+H) 222,1

Zu 7,3 ml (57,36 mmol) N-Ethylanilin in 250 ml Acetonitril wurden unter Argon Schutzatmosphäre 15,5 ml (63,51 mmol) N,O-Bis(Trimethylsilyl)acetamid gegeben und 3 h bei 50°C gerührt. Anschließend wurde bei 20°C eine Lösung des obigen Lactons (10,7 g, 48,37 mmol) gelöst in 250 ml Acetonitril zugegeben und 17 h unter Rückfluss erhitzt. Nach Entfernung des Lösungsmittels wurde der Rückstand mit gesättigter Natriumcarbonat Lösung versetzt wobei der pH Wert der Lösung 9 nicht überschritt. Die wässrige Suspension wurde mit wenig Diethylether gewaschen und anschließend mit konz. Salzsäure auf einen pH-Wert von 6 bis 7 acidifiziert und mit NaHPO₄ Puffer ein pH-Wert von 5 eingestellt. Die wässrige Lösung wurde dann mehrmals mit Essigester extrahiert. Nach Evaporierung der Lösungsmittel erhielt man das gewünschte Produkt in einer Ausbeute von 45% (7,4 g). Summenformel C₁₉H₂₂N₂O₄; M.W. = 342,4; MS (M+H) 343,2
Zu 75 ml Methanol wurden bei -10°C 6,5 ml (89,1 mmol) Thionylchlorid getropft und 30 min gerührt. Anschließend wurde in 75 ml Methanol gelöste L-2-Aminoethyl-3-phenylaminopropionsäure 8,6 g (25,12 mmol) zugegeben, 30 Minuten bei -10°C und weitere 3 h bei Raumtemperatur gerührt. Nach der Evaporierung der Lösungsmittel wurde der Rückstand in Essigester aufgenommen und mit Natriumcarbonat Lösung gewaschen. Nach Evaporierung des Lösungsmittels und Reinigung mittels Flash-Chromatographie (n-Heptan/Essigester 7:3) wurde 4,43 g (50% Ausbeute) von L-2-Aminoethyl-3-phenylaminopropionsäure-methylester erhalten. Summenformel C₂₀H₂₄N₂O₄; M.W. = 356,4; MS (M+H) 357,3

Zur Abspaltung der Schutzgruppe löste man 4,4 g (12,35 mmol) des Z-geschützte Derivates in 500 ml Methanol, fügte unter Inertgas 100 mg Katalysator (10% Pd(OH)₂-C) zu, und leitete bis zur vollständigen Abspaltung der Z-Schutzgruppe Wasserstoff ein. Nach Abfiltrieren des Katalysators und Eindampfen des Filtrates erhielt man 2,8 g L-2-Aminoethyl-3-phenylamino-propionsäure (quantitativ).
Summenformel C₁₂H₁₈N₂O₂; M.W. = 223,3; MS (M+H) 223,1

### Verfahrensschritt b)

0,63 g (2,64 mmol) 2-Pyridin-4-yl-1 H-indol-5-carbonsäure hergestellt wie in Verfahrensvariante B) wurden in 150 ml DMF suspendiert und nacheinander mit 1,01 g (3,08 mmol) TOTU und 0,63 ml (3,71 mmol) Ethyldiisopropylamin versetzt. Man rührte 20 min. bei RT und gab zu der entstandenen klaren Lösung 0,73 g (3,28 mmol) (S)-2-Amino-3-phenylaminethylpropionsäuremethylester hergestellt gemäß a) hinzu. Nach 15 h Rühren engte man unter vermindertem Druck ein und isolierte den Methylester der Titelverbindung durch Flash-Chromatographie an Kieselgel (DCM:MeOH= 19:1). Ausbeute: 0,44 g, Summenformel C₂₆H₂₆N₄O₃; M.W. = 442,2; MS (M+H) 443,3.

0,22 g ( 0,497 mmol) des so erhaltenen Methylesters wurde in 100 ml Methanol gelöst auf 0 °C gekühlt und dann 1.5 h Ammoniak eingeleitet. Man lies die Lösung über Nacht bei Raumtemperatur stehen und evaporierte anschließend das Methanol. Das Rohprodukt wurde mittels Flash-Chromatographie an Kieselgel (DCM:MeOH= 19:1) gereinigt. Ausbeute: 0,096g (45,2%) C₂₅H₂₅N₅O₂;M.W. = 427,2; MS (M+H) 428,3;

In der nachfolgenden Tabelle 1 wurden die Verbindungen analog zu den Verfahren A) bis D) hergestellt.

**Tabelle 1:**

| Beispiel | Struktur | Summenformel | MS (M+ H) | Bem. |
|---|---|---|---|---|
| 1 | 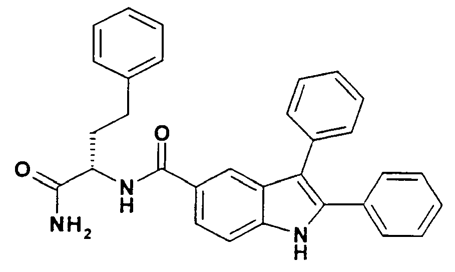 | M.W. = 473,58 | 474,2 | Verf.: A) ; |
| | | C₃₁H₂₇N₃O₂ | | Verf.:C) |
| 2 | 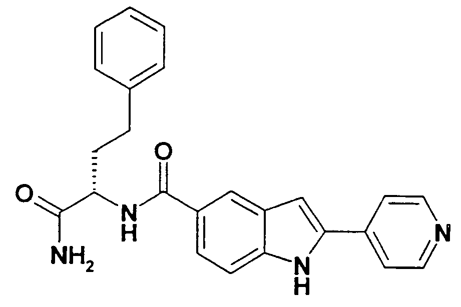 | M.W. = 398,46 | 399,3 | Verf.: B) |
| | | C₂₄H₂₂N₄O₂ | | Verf.:C) |
| 3 | 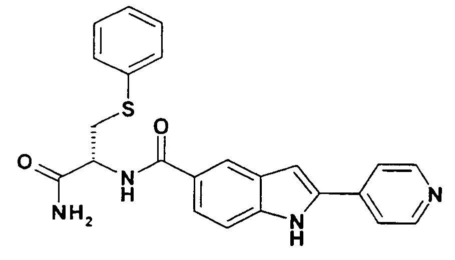 | M.W. = 416,50 | 417,1 | Verf.: A) |
| | | C₂₃H₂₀N₄O₂S | | Verf.:D) |
| 4 | 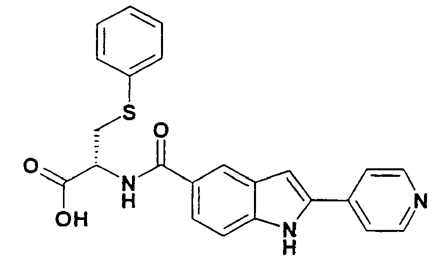 | M.W. = 417,9 | 418,1 | |
| | | C₂₃H₁₉N₃O₃S | | Verf.: B) |
| | | | | Verf.:C) |
| 5 | 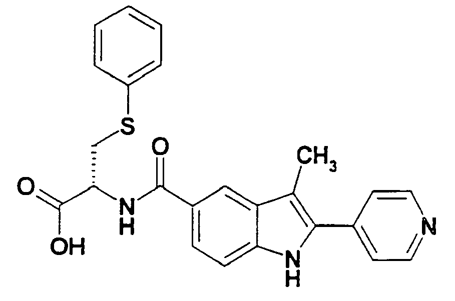 | M.W. = 431,51 | 432,1 | |
| | | C24H₂₁N₃O₃S | | Verf.: B) |
| | | | | Verf.:C) |
| 6 | 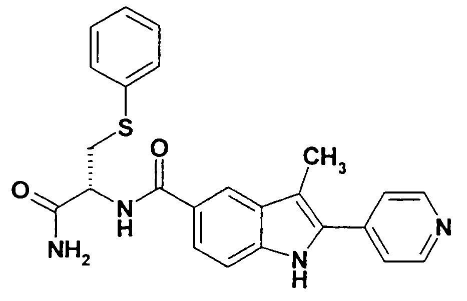 | M.W. = 430,53 | 431,2 | |
| | | C₂₄H₂₂N₄O₂S | | Verf.: B) |
| | | | | Verf.:C) |
| 7 | 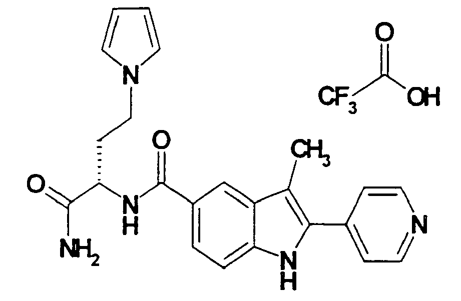 | M.W. = 516,47 | 403,2 | |
| | | C₂₃H₂₂N₄O₃ * | | Verf.: B) |
| | | C₂HF₃O₂ | | Verf.:C) |
| 8 | 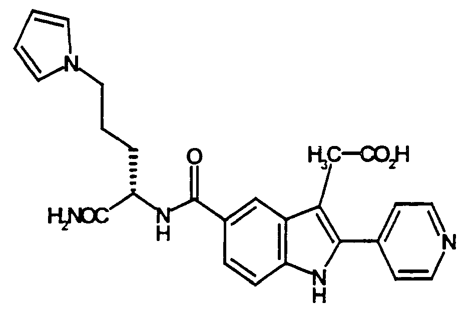 | M.W. = 475,50 | 416,5 | |
| | | C₂₄H₂₅N₅O₂ * | | Verf.: B) |
| | | C₂H₄O₂ | | Verf.:C) |
| 9 | 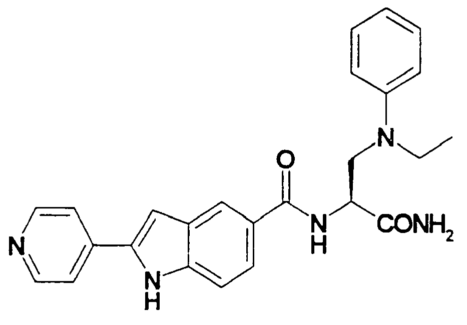 | M.W. = 427,2; | 428,3 | |
| | | C₂₅H₂₅N₅O₂; | | |

| | | | | |
|---|---|---|---|---|
| Bem. = Bemerkungen Verf. = Verfahrensvariante Pharmakologische Beispiele lκB-Kinase ELISA: | | | | |

Die Aktivität der lκB-Kinase wurde mit einem ELISA, bestehend aus einem biotiniliertem Substratpeptid, welches die Aminosäuresequenz im Protein lκB von Serine 32 bis 36 enthält, und einem spezifischen poly- oder monoklonalen Antikörper (z.B. von New England Biolabs, Beverly, MA, USA, Kat.: 9240), der nur an die phosphorylierte Form des Peptids lκB bindet, bestimmt. Dieser Komplex wurde an einer antikörperbindenden Platte (Protein A beschichtet) immobilisiert und mit einem Konjugat aus einem biotinbindendem Protein und HRP (z.B. Streptavidin-HRP) detektiert. Die Aktivität konnte an Hand einer Standardkurve mit Substratphosphopeptid quantifiziert werden.

### Durchführung:

Zur Gewinnung des Kinasekomplexes wurden 10 ml HeLa S3-Zellextrakt S100 mit 40 ml 50mM HEPES, pH 7,5, verdünnt, auf 40% Ammoniumsulfat gebracht und auf Eis 30 Minuten inkubiert. Das präzipitierte Pellet wurde in 5 ml SEC Puffer (50 mM HEPES, pH 7,5, 1 mM DTT, 0,5 mM EDTA, 10 mM 2-Glyzerophosphat) gelöst, bei 20,000 x g für 15 Minuten zentrifugiert und durch einen 0,22 µm Filter filtriert. Die Probe wurde auf eine 320 ml Superose-6 FPLC Säule (Amersham Pharmacia Biotech AB, Uppsala, Schweden) aufgetragen, die mit SEC Puffer äquilibriert war und mit einer Flußrate von 2 ml/min bei 4 °C betrieben wurde. Die Fraktionen, die bei der Laufzeit des 670 kDa Molekulargewichtstandards lagen, wurden für die Aktivierung vereinigt. Die Aktivierung wurde durch eine 45-minütige Inkubation mit 100 nM MEKK1Δ, 250 µM MgATP, 10 mM MgCl₂, 5 mM Dithiothreitol (DTT), 10 mM 2-Glyzerophosphat, 2,5 µM Microcystin-LR bei 37 °C erreicht. Das aktivierte Enzym wurde bei -80 °C gelagert.
Die in DMSO gelösten Testsubstanzen (2 µl) wurden 30 Minuten bei 25°C mit 43 µl aktiviertem Enzym (1:25 verdünnt in Reaktionspuffer 50 mM HEPES, pH 7,5, 10 mM MgCl₂, 5 mM DTT, 10 mM ß-Glycerophosphat, 2,5 µM Microcystin-LR) vorinkubiert. Dann wurden 5 µl Substratpeptid (Biotin-(CH₂)₆-DRHDSGLDSMKD-CONH₂) (200 µM) dazugegeben, eine Stunde inkubiert und mit 150 µl 50 mM HEPES pH 7,5, 0.1% BSA, 50 mM EDTA, Antikörper [1:200] abgestoppt. 100 µl des abgestoppten Reaktionsgemisches bzw. einer Standardphosphopeptidverdünnungsreihe (Biotin-(CH₂)₆-DRHDS[PO₃]GLDSMKD-CONH₂) wurden dann in eine Protein-A Platte (Pierce Chemical Co., Rockford, IL, USA) überführt und 2 Stunden unter Schütteln inkubiert. Nach 3 Waschschritten mit PBS, wurden 100 µl 0,5 µg/ml Streptavidin-HRP (horseradish peroxidase) (verdünnt in 50 mM HEPES/ 0,1% BSA) für 30 Minuten hinzugegeben. Nach 5 Waschschritten mit PBS, wurden 100 µL TMB-Substrat (Kirkegaard & Perry Laboratories, Gaithersburg, MD, USA) hinzugegeben und die Farbentwicklung durch Zugabe von 100 µL 0,18 M Schwefelsäure abgestoppt. Die Absorption wurde bei 450 nm gemessen. Die Standardkurve wurde durch lineare Regression entsprechend einer 4-Parameter Dosis-Wirkungsbeziehung erzeugt. An Hand dieser Standardkurve wurde die Enzymaktivität bzw. deren Inhibition durch die Testsubstanzen quantifiziert.
Methode PKA, PKC, CK II
cAMP-abhängige Proteinkinase (PKA), Proteinkinase C (PKC) und Caseinkinase II (CK II) wurden mit den entsprechenden Testkits von Upstate Biotechnologie gemäß der Vorschrift des Hersteller bei einer ATP-Konzentration von 50 µM bestimmt. Abweichend wurden statt Phosphocellulosefilter Multi-Screen-Platten (Millipore; Phosphocellulose MS-PH, Kat. MAPHNOB10) mit dem entsprechenden Absaugsystem verwendet. Die Platten wurden anschließend in einem Wallac MicroBeta Szintillationszähler vermessen. Es wurde jeweils 100 µM der Testsubstanz eingesetzt.
Jede Substanz wurde in Doppelbestimmung getestet. Von den Mittelwerten (Enzym mit und ohne Substanzen) wurde der Mittelwert des Blanks (ohne Enzym) subtrahiert und die % Inhibition errechnet. IC₅₀-Berechnungen wurden mit dem Softwarepaket GraFit 3.0 durchgeführt. Die nachfolgende Tabelle 2 zeigt die Ergebnisse.

**Tabelle 2: Kinaseinhibition bei einer Substanzkonzentration von 100 µM oder IC₅₀ in µM**

| Beispiel Nummer | lκB-Kinase IC₅₀ | PKA %-Hemmung | PKC %-Hemmung | CK II %-Hemmung |
|---|---|---|---|---|
| 1 | 32 | n.b. | n.b. | n.b. |
| 2 | 0,61 | 24 | 15 | 35 |
| 3 | 0,55 | 35 | 39 | 37 |
| 4 | 0,50 | 42 | 33 | 47 |
| 5 | 1,8 | 55 | 8 | 27 |
| 6 | 4,9 | 60 | 58 | 39 |
| 7 | 3,0 | n.b. | n.b. | 18 |
| 9 | 1,0 | 0 | 23 | 0 |

| | | | | |
|---|---|---|---|---|
| n.b. bedeutet nicht bestimmt | | | | |

## Patentansprüche

1. Verbindung Formel I 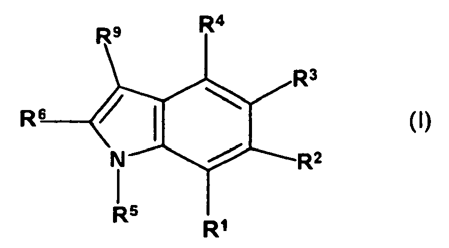 und/oder eine stereoisomere Form der Verbindung der Formel I und/oder ein physiologisch verträgliches Salz der Verbindung der Formel I, wobei
R³ für einen Rest der Formel II steht, 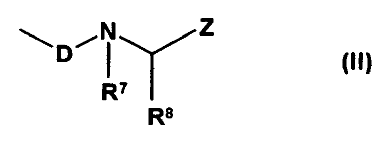 worin
D für -C(O)- steht,
R⁷ für Wasserstoffatom oder -(C₁-C₄)-Alkyl steht,
R⁸ für
1. -(C₁-C₄)-Alkyl, worin Alkyl gerade oder verzweigt ist und ein- oder zweifach unabhängig voneinander substituiert ist durch
1.1 Heteroaryl mit 5 bis 14 Ringgliedern oder Heterocyclus mit 5 bis 12 Ringgliedern, worin Heteroaryl und Heterocyclus ausgewählt wurden aus der Gruppe Pyrrol, Pyridin, Pyrazin, Furan, Thiophen, Imidazol, Pyrazol, Oxazol, Isoxazol, Thiazol, Isothiazol, Tetrazol, Triazolone, 1,2,3,5-Oxathiadiazol-2-Oxide, Oxadiazolone, Isoxazolone, Oxadiazolidindione, Triazole, welche durch F, -CN, -CF₃ oder -C(O)-O-(C₁-C₄)-Alkyl substituiert sind, 3-Hydroxypyrro-2,4-dione, 5-Oxo-1,2,4-Thiadiazole, Pyrimidin, Indol, Isoindol, Indazol, Phthalazin, Chinolin, Isochinolin, Chinoxalin, Chinazolin, Cinnolin, -Carbolin und benzanellierte, cyclopenta-, cyclohexa- und cyclohepta-anellierte Derivate dieser Heterocyclen ableiten,
1.2 -O-R¹⁰,
1.3 -S(O)ₓ-R¹⁰, worin x die ganze Zahl Null, 1 oder 2 ist,
1.4 -N(R¹⁰)₂,
1.5 Rest der Formel 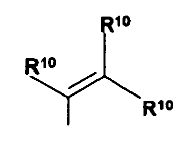 oder
1.6 Rest der Formel 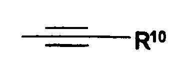 oder
2. für den charakteristischen Rest einer Aminosäure steht aus der Gruppe Glycin, Alanin, Valin, Leucin, Isoleucin, Phenylalanin, Tyrosin, Serin, Tryptophan, Threonin, Cystein, Methionin, Asparagin, Glutamin, Lysin, Histidin, Arginin, Glutaminsäure, Asparaginsäure, 2-Aminoadipinsäure, 2-Aminoisobutter-säure, 2-Aminobuttersäure, 2,3-Diamino-propionsäure, 2,4-Diaminobuttersäure, 1,2,3,4-Tetrahydroisochinolin-1-carbonsäure, 1,2,3,4-Tetrahydroisochinolin-3-carbonsäure, 2-Aminopimelinsäure, Phenylglycin, 3-(2-Thienyl)-alanin, 3-(3-Thienyl)-alanin, 2-(2-Thienyl)-glycin, 2-Amino-heptansäure, Pipecolinsäure, Hydroxylysin, Sarkosin, N-Methylisoleucin, 6-N-Methyl-lysin, N-Methylvalin, Norvalin, Norleucin, Omithin, allo-Isoleucin, allo-Threonin, allo-Hydroxylysin, 4-Hydroxyprolin, 3-Hydroxyprolin, 3-(2-Naphtyl)-alanin, 3-(1-Naphtyl-alanin), Homophenylalanin, Homocystein, Homocysteinsäure, Homotryptophan, Cysteinsäure, 3-(2-Pyridyl)-alanin, 3-(3-Pyridyl)-alanin, 3-(4-Pyridyl)-alanin, 2-Amino-3-phenylaminopropionsäure, 2-Amino-3-phenylaminoethylpropionsäure, Phosphinothricin, 4-Fluorphenylalanin, 3-Fluorphenyl-alanin, 2-Fluorphenylalanin, 4-Chlorphenylalanin, 4-Nitrophenylalanin, 4-Aminophenylalanin, Citrullin, Cyclohexylalanin, 5-Fluortryptophan, 5-Methoxytryptophan, Methionin-Sulfon, Methionin-Sulfoxid und -NH-NR¹⁰-C(O)N(R¹⁰)₂; steht,
R⁹ für
1. R⁸,
2. -(C₁-C₄)-Alkyl, worin Alkyl gerade oder verzweigt ist und unabhängig voneinander ein-, zwei- oder dreifach substituiert ist durch
2.1 Aryl, worin Aryl für einen Rest aus der Gruppe Phenyl, Naphthyl, Biphenylyl, Anthryl oder Fluorenyl steht und der Arylrest unsubstituiert ist oder einfach, zweifach oder dreifach, durch gleiche oder verschiedene Reste aus der Reihe -(C₁-C₈)-Alkyl, -(C₁-C₈)-Alkoxy, Halogen, Nitro, Amino, Trifluormethyl, Hydroxy, Hydroxy-(C₁-C₄)-alkyl wie Hydroxymethyl oder 1-Hydroxyethyl oder 2-Hydroxyethyl, Methylendioxy, Ethylendioxy, Formyl, Acetyl, Cyano, Hydroxycarbonyl, Aminocarbonyl, -(C₁-C₄)-Alkoxycarbonyl, Phenyl, Phenoxy, Benzyl, Benzyloxy oder Tetrazolyl substituiert ist,
2.2 Halogen,
2.3 -CN oder
2.4 -CF₃ ,
3. Aryl, worin Aryl wie oben definiert ist und unsubstituiert oder wie oben substituiert ist, steht,
R¹⁰ für
a) Wasserstoffatom,
b) -(C₁-C₆)-Alkyl, worin Alkyl unsubstituiert oder ein- bis dreifach unabhängig voneinander substituiert ist durch
1. Aryl, worin Aryl wie oben definiert ist,
2. Heteroaryl mit 5 bis 14 Ringgliedem, worin Heteroaryl wie oben definiert ist,
3. Heterocyclus mit 5 bis 12 Ringgliedem, worin Heterocyclus wie oben definiert ist,
4. Halogen,
5. -N-(C₁-C₆)ₙ-Alkyl, worin n die ganze Zahl Null, 1 oder 2 bedeutet und Alkyl unsubstituiert oder ein-, zwei- oder dreifach unabhängig voneinander durch Halogen oder durch -C(O)-OH substituiert ist, oder
6. -C(O)-OH,
c) Aryl, worin Aryl wie oben definiert ist,
d) Heteroaryl mit 5 bis 14 Ringgliedern, worin Heteroaryl wie oben definiert ist, oder
e) Heterocyclus mit 5 bis 12 Ringgliedern, worin Heterocyclus wie oben definiert ist, steht und
für den Fall des (R¹⁰)₂ hat R¹⁰ unabhängig voneinander die Bedeutung von a) bis e),
Z für
1. 1,3,4-Oxadiazol, worin 1,3,4-Oxadiazol unsubstituiert oder ein- bis dreifach substituiert ist durch -NH₂, OH oder -(C₁-C₄)-Alkyl oder
2. -C(O)-R¹¹ steht, worin
R¹¹ für
1. -O-R¹⁰ oder
2. -N(R¹⁰)₂ steht, oder
R⁷und R⁸ bilden zusammen mit dem Stickstoff- und Kohlenstoffatom an das sie jeweils gebunden sind einen Ring der Formel IIa 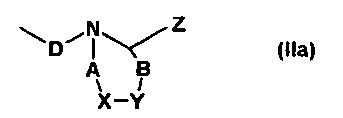 aus der Gruppe Pyrrol, Pyrrolin, Pyrrolidin, Pyridin, Piperidin, Piperylen, Pyridazin, Pyrimidin, Pyrazin, Piperazin, Pyrazol, Imidazol, Pyrazolin, Imidazolin, Pyrazolidin, Imidazolidin, Oxazol, Tetrazol, 1,2,3,5-Oxathiadiazol-2-Oxide, Triazolone, Oxadiazolone, Isoxazolone, Oxadiazolidindione, Triazole, welche unsubstituiert oder durch F, -CN, -CF₃ oder C(O)-O-(C₁-C₄)-Alkyl substituert sind, 3-Hydroxypyrro-2,4-dione, 5-Oxo-1,2,4-Thiadiazole, Isoxazol, 2-Isoxazolidin, Isoxazolidin, Morpholin, Isothiazol, Thiazol, Isothiazolidin, Thiomorpholin, Indazol, Thiadiazol, Benzimidazol, Chinolin, Triazol, Phthalazin, Chinazolin, Chinoxalin, Purin, Pteridin, Indol, Isochinolin, Tetrahydrochinolin und Tetrahydroisochinolin, oder
R⁸ und Z bilden zusammen mit den Kohlenstoffatomen an das sie jeweils gebunden sind einen Ring der Formel IIc 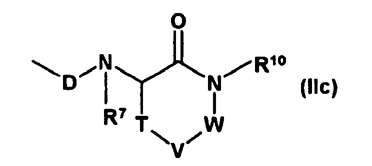 aus der Gruppe Pyrrol, Pyrrolin, Pyrrolidin, Pyridin, Piperidin, Pyrazolin, Phthalazin, Piperylen, Pyridazin, Pyrimidin, Pyrazin, Piperazin, Pyrazol, Imidazol, 1,3,4-Oxadiazol, Imidazolin, Pyrazolidin, Imidazolidin, Oxazol, Isoxazol, 2-Isoxazolidin, Isoxazolidin, Morpholin, Isothiazol, Thiazol, Isothiazolidin, Thiomorpholin, Indazol. Thiadiazol, Benzimidazol, Chinolin, Triazol, Tetrazol, 1,2,3,5-Oxathiadiazol-2-Oxide, Oxadiazolone, Isoxazolone, Triazolone, Oxadiazolidindione, Triazole, welche unsubstituiert oder durch F, -CN, -CF₃ oder -C(O)-O-(C₁-C₄)-Alkyl substituiert sind, 3-Hydroxypyrro-2,4-dione, 5-Oxo-1,2,4-Thiadiazole, Chinazolin, Chinoxalin, Purin, Indol, Pteridin, Tetrahydrochinolin, Tetrahydroisochinolin und Isochinolin, und
R¹, R² und R⁴ für Wasserstoffatom stehen,
R⁵ für Wasserstoffatom steht und
R⁶ für
1. Phenyl, ein- oder zweifach unabhängig voneinander substituiert durch
1.1 -CN,
1.2 -CF₃,
1.3 Halogen,
1.4 -O-R¹⁰,
1.5 -N(R¹⁰)₂,
1.6 -NH-C(O)-R¹¹,
1.7 -S(O)ₓ -R¹⁰, worin x die ganze Zahl Null, 1 oder 2 ist,
1.8 -C(O)-R¹¹ oder
1.9 -(C₁-C₄)-Alkyl-NH₂,
2. Heteroaryl mit 5 bis 14 Ringgliedern, worin Heteroaryl wie oben definiert ist und unsubstituiert oder ein-, zwei- oder dreifach unabhängig voneinander substituiert ist durch die oben unter 1.1 bis 1.9 definierten Substituenten oder
3. Heterocyclus mit 5 bis 12 Ringgliedem, worin Heterocyclus wie oben definiert ist und unsubstituiert oder ein-, zwei- oder dreifach unabhängig voneinander substituiert ist durch die oben unter 1.1 bis 1.9 definierten Substituenten, steht.

2. Verbindung der Formel I gemäß Anspruch 1, **dadurch gekennzeichnet, dass**
R³ für einen Rest der Formel II steht, worin
D für -C(O)- steht,
R⁷ für Wasserstoffatom steht,
Z für -C(O)-OH oder -C(O)-NH₂ steht.
R⁸ für
1. -(C₁-C₄)-Alkyl, worin Alkyl gerade oder verzweigt ist und ein- oder zweifach unabhängig voneinander substituiert ist durch
1.1 -S(O)-R¹⁰, wobei R¹⁰ wie unten definiert ist,
1.2 -N(R¹⁰)₂, wobei R¹⁰ wie unten definiert ist, oder
1.3 Pyrrol, oder
2. für den charakteristischen Rest eine Aminosäure aus der Gruppe Histidin, Tryptophan, Serin, Threonin, Cystein, Methionin, Asparagin, Glutamin, Lysin, Arginin, Glutaminsäure und Asparaginsäure steht,
R⁹ für
1. Wasserstoffatom,
2. -(C₁-C₄)-Alkyl, worin Alkyl gerade oder verzweigt ist und unabhängig voneinander ein-, zwei- oder dreifach substituiert ist durch -C(O)-OH, -OH oder -C(O)-NH₂, oder
3. Phenyl, worin Phenyl unsubstituiert oder ein bis dreifach unabhängig voneinander substituiert ist durch Halogen oder -(C₁-C₄)-Alkyl, steht
R¹⁰ für
a) Wasserstoffatom,
b) -(C₁-C₆)-Alkyl, worin Alkyl unsubstituiert oder ein bis dreifach unabhängig voneinander substituiert ist durch Halogen,
c) Phenyl, worin Phenyl unsubstituiert oder ein bis dreifach unabhängig voneinander substituiert ist durch Halogen oder -(C₁-C₄)-Alkyl,
R¹ R² und R⁴ für Wasserstoffatom stehen,
R⁵ für Wasserstoffatom steht, und
R⁶ für Phenyl oder Pyridin steht.

3. Verfahren zur Herstellung der Verbindung der Formel I gemäß der Ansprüche 1 oder 2, **dadurch gekennzeichnet, daß** man
a) eine Verbindung der Formel IV, 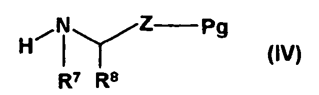 worin Pg ein geeignete Schutzgruppe (z.B. Methylester), eine Amidgruppe oder eine Hydroxy-Gruppe darstellt und Z, R⁷ und R⁸ wie in Formel I definiert sind, mit 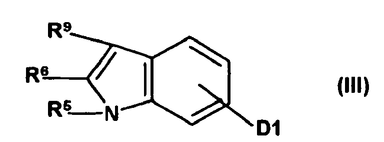 einem Säurechlorid oder einem aktivierten Ester der Verbindung der Formel III, wobei D1 -COOH oder Sulfonylhalogen bedeutet und R⁵, R⁶ und R⁹ wie in Formel I definiert sind, in Gegenwart einer Base oder gegebenenfalls eines wasserentziehenden Mittels in Lösung umsetzt und nach Abspaltung der Schutzgruppe in eine Verbindung der Formel I überführt, oder
b) eine Verbindung der Formel IVa, 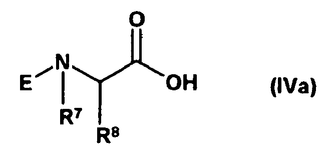 worin R⁷ und R⁸ wie in Formel I definiert sind und E eine N-Aminoschutzgruppe darstellt, mit ihrer Carboxylgruppe über eine Zwischenkette L an ein polymeres Harz der allgemeinen Formel PS ankoppelt, wobei eine Verbindung der Formel V 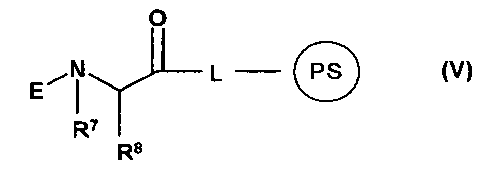 entsteht, die nach selektiver Abspaltung der Schutzgruppe E mit einer Verbindung der Formel III, wobei R⁵, R⁶ und R⁹ wie in Formel I definiert sind, in Gegenwart einer Base oder gegebenenfalls eines wasserentziehenden Mittels zu einer Verbindung der Formel VI 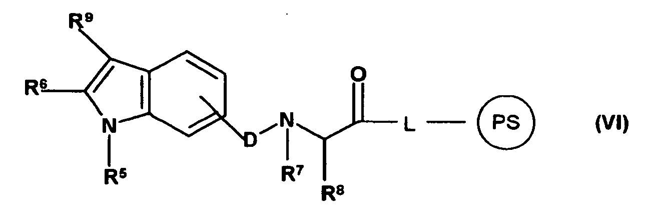 umsetzt, und die Verbindung der Formel VI nach Abspaltung vom Trägermaterial in eine Verbindung der Formel I überführt, oder
c) eine Verbindung der Formel I in ein physiologisch verträgliches Salz überführt.

4. Arzneimittel, **gekennzeichnet durch** einen wirksamen Gehalt an mindestens einer Verbindung der Formel I gemäß der Ansprüche 1 oder 2 zusammen mit einem pharmazeutisch geeigneten und physiologisch verträglichen Trägerstoff, Zusatzstoff und/oder anderen Wirk- und Hilfsstoffen.

5. Verwendung von mindestens einer Verbindung der Formel I gemäß der Ansprüche 1 oder 2, zur Herstellung von Arzneimitteln zur Prophylaxe und Therapie von Erkrankungen, an deren Verlauf eine verstärkte Aktivität von NFκB beteiligt ist.

6. Verwendung gemäß Anspruch 5, für die Behandlung von chronischen Erkrankungen des Bewegungsapparates wie entzündliche, immunologisch oder stoffwechselbedingte akute und chronische Arthritiden, Arthropathien, rheumatoide Arthritis, oder degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen oder Erkrankungen des Bindegewebes wie Kollagenosen und Periodontalerkrankungen, Myalgien und Störungen des Knochenstoffwechsels, oder Erkrankungen, die durch eine Überexpression von Tumor Nekrose Faktor alpha (TNFα) oder erhöhte Konzentration an TNFα bedingt sind wie Kachexie, Mutiple Sklerose, Schädel-Him Trauma, Morbus Crohn und Darmgeschwüre, oder Atherosklerose, Stenosen, Ulceration, Alzheimers Erkrankungen, Muskelabbau, Krebserkrankungen (Potenzierung von Cytotoxica-Therapien), Herzinfarkt, Gicht, Sepsis, septischer Schock, endotoxischer Schock, virale Infektionen wie Grippe, Hepatitis, HIV-Infektionen, AIDS, oder durch Adenoviren oder Herpesviren verursachte Erkrankungen, parasitische Infektionen wie Malaria oder Lepra, Pilz- oder Hefeinfektionen, Gehirnhautentzündungen, chronische entzündliche Lungenerkrankungen wie chronische Bronchitis oder Asthma, acute respiratory distress syndrome, akute Synovitis, Tuberkulose, Psoriasis, Diabetes, Behandlung von akuten oder chronischen Abstoßungsreaktionen des Organempfängers gegen das verpflanzte Organ, chronische Graft-versus-Host-Erkrankungen und entzündliche Gefäßerkrankungen.

7. Verfahren zur Herstellung eines Arzneimittels, **dadurch gekennzeichnet, daß** man mindestens eine Verbindung der Formel I gemäß der Ansprüche 1 oder 2 mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

## Claims

1. A compound of the formula I 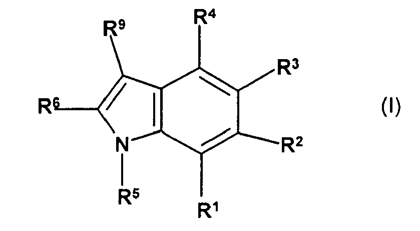 and/or a stereoisomeric form of the compound of the formula I and/or a physiologically acceptable salt of the compound of the formula I, where R³ is a radical of the formula II 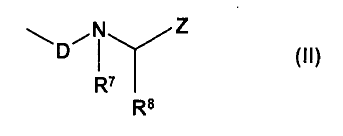 in which
D is -C (O) -,
R⁷ is hydrogen or - (C₁-C₄) -alkyl,
R⁸ is
1. - (C₁-C₄) -alkyl, where alkyl is straight-chain or branched and is mono- or disubstituted, independently of one another, by
1.1 heteroaryl having 5 to 14 ring members or heterocycle having 5 to 12 ring members, where heteroaryl and heterocycle were selected from the group consisting of pyrrole, pyridine, pyrazine, furan, thiophene, imidazole, pyrazole, oxazole, isoxazole, thiazole, isothiazole, tetrazole, triazolones, 1,2,3,5-oxathiadiazole 2-oxides, oxadiazolones, isoxazolones, oxadiazolidinediones, triazoles, which are substituted by F, -CN, -CF3 or -C(O)-O-(C1-C4)-alkyl, 3-hydroxypyrrole-2,4-diones, 5-oxo-1,2,4-thiadiazoles, pyrimidine, indole, isoindole, indazole, phthalazine, quinoline, isoquinoline, quinoxaline, quinazoline, cinnoline, carboline and benzo-fused, cyclopenta-, cyclohexa- and cyclohepta-fused derivatives of these heterocycles,
1.2 -O-R¹⁰,
1.3 -S(O)ₓ-R¹⁰, where x is the integer zero, 1 or 2,
1.4 -N (R¹⁰) ₂,
1.5 radical of the formula 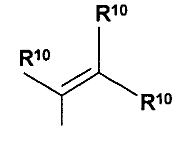 or
1.6 radical of the formula 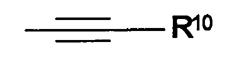 or
2. is the characteristic radical of an amino acid from the group consisting of glycine, alanine, valine, leucine, isoleucine, phenylalanine, tyrosine, serine, tryptophan, threonine, cysteine, methionine, asparagine, glutamine, lysine, histidine, arginine, glutamic acid, aspartic acid, 2-aminoadipic acid, 2-aminoisobutyric acid, 2-aminobutyric acid, 2,3-diaminopropionic acid, 2,4-diaminobutyric acid, 1,2,3,4-tetrahydroisoquinoline-1-carboxylic acid, 1,2,3,4-tetrahydroisoquinoline-3-carboxylic acid, 2-aminopimelic acid, phenylglycine, 3-(2-thienyl)alanine, 3-(3-thienyl)alanine, 2-(2-thienyl)glycine, 2-aminoheptanoic acid, pipecolic acid, hydroxylysine, sarcosine, N-methylisoleucine, 6-N-methyllysine, N-methylvaline, norvaline, norleucine, ornithine, allo-isoleucine, allothreonine, allo-hydroxylysine, 4-hydroxyproline, 3-hydroxyproline, 3-(2-naphthyl)alanine, 3-(1-naphthylalanine), homophenylalanine, homocysteine, homocysteic acid, homotryptophan, cysteic acid, 3-(2-pyridyl)alanine, 3-(3-pyridyl)alanine, 3-(4-pyridyl)alanine, 2-amino-3-phenylaminopropionic acid, 2-amino-3-phenylaminoethylpropionic acid, phosphinothricine, 4-fluorophenylalanine, 3-fluorophenylalanine, 2-fluorophenylalanine, 4-chlorophenylalanine, 4-nitrophenylalanine, 4-aminophenylalanine, citrulline, cyclohexylalanine, 5-fluorotryptophan, 5-methoxytryptophan, methionine sulfone, methionine sulfoxide and -NH-NR¹⁰-CON (R¹⁰) ₂,
R⁹ is
1. R⁸,
2. -(C₁-C₄)-alkyl, where alkyl is straight-chain or branched and is, independently of one another, mono-, di- or trisubstituted by
2.1 aryl, where aryl is a radical from the group consisting of phenyl, naphthyl, biphenylyl, anthryl or fluorenyl and the aryl radical is unsubstituted or monosubstituted, disubstituted or trisubstituted, by identical or different radicals from the group consisting of (C₁-C₈)-alkyl, (C₁-C₈)-alkoxy, halogen, nitro, amino, trifluoromethyl, hydroxyl, hydroxy- (C₁-C₄) -alkyl such as hydroxymethyl or 1-hydroxyethyl or 2-hydroxyethyl, methylenedioxy, ethylenedioxy, formyl, acetyl, cyano, hydroxycarbonyl, aminocarbonyl, (C₁-C₄) -alkoxycarbonyl, phenyl, phenoxy, benzyl, benzyloxy or tetrazolyl,
2.2 halogen,
2.3 -CN or
2.4 -CF₃,
3. aryl, where aryl is as defined above and is unsubstituted or substituted as above,
R¹⁰ is
a) hydrogen,
b) - (C₁-C₆) -alkyl, where alkyl is unsubstituted or mono- to trisubstituted, independently of one another, by
1. aryl, in which aryl is as defined above,
2. heteroaryl having 5 to 14 ring members, in which heteroaryl is as defined above,
3. heterocycle having 5 to 12 ring members, in which heterocycle is as defined above,
4. halogen,
5. -N- (C₁-C₆) ₙ-alkyl, where n is the integer zero, 1 or 2 and alkyl is unsubstituted or mono-, di- or trisubstituted, independently of one another, by halogen or by -C(O)-OH, or
6. -C(O)-OH,
c) aryl, in which aryl is as defined above,
d) heteroaryl having 5 to 14 ring members, in which heteroaryl is as defined above, or
e) heterocycle having 5 to 12 ring members, in which heterocycle is as defined above, and,
in the case of (R¹⁰) ₂, R¹⁰, independently of one another, has the meaning of a) to e),
Z is
1. 1,3,4-oxadiazole, where 1,3,4-oxadiazole is unsubstituted or mono- to trisubstituted by -NH₂, OH or -(C₁-C₄)-alkyl or
2. -C (O) -R¹¹, in which
R¹¹ is
1. -O-R¹⁰ or
2. -N (R¹⁰) ₂, or
R⁷ and R⁸ form, together with the nitrogen atom and carbon atom to which they are each bonded, a ring of the formula IIa 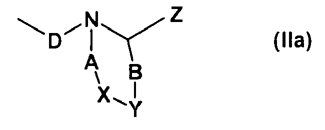 selected from the group consisting of pyrrole, pyrroline, pyrrolidine, pyridine, piperidine, piperylene, pyridazine, pyrimidine, pyrazine, piperazine, pyrazole, imidazole, pyrazoline, imidazoline, pyrazolidine, imidazolidine, oxazole, tetrazole, 1,2,3,5-oxathiadiazole 2-oxides, triazolones, oxadiazolones, isoxazolones, oxadiazolidinediones, triazoles, which are unsubstituted or substituted by F, -CN, -CF₃ or C(O)-O-(C₁-C₄)-alkyl, 3-hydroxypyrrole-2,4-diones, 5-oxo-1,2,4-thiadiazoles, isoxazoles, 2-isoxazolidine, isoxazolidine, morpholine, isothiazole, thiazole, isothiazolidine, thiomorpholine, indazole, thiadiazole, benzimidazole, quinoline, triazole, phthalazine, quinazoline, quinoxaline, purine, pteridine, indole, isoquinoline, tetrahydroquinoline and tetrahydroisoquinoline, or
R⁸ and Z form, together with the carbon atoms to which they are each bonded, a ring of the formula IIc 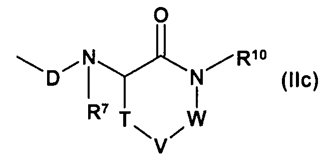 selected from the group consisting of pyrrole, pyrroline, pyrrolidine, pyridine, piperidine, pyrazoline, phthalazine, piperylene, pyridazine, pyrimidine, pyrazine, piperazine, pyrazole, imidazole, 1,3,4-oxadiazole, imidazoline, pyrazolidine, imidazolidine, oxazole, isoxazole, 2-isoxazolidine, isoxazolidine, morpholine, isothiazole, thiazole, isothiazolidine, thiomorpholine, indazole, thiadiazole, benzimidazole, quinoline, triazole, tetrazole, 1,2,3,5-oxathiadiazole 2-oxides, oxadiazolones, isoxazolones, triazolones, oxadiazolidindiones, triazoles, which are unsubstituted or substituted by F, -CN, -CF₃ or -C(O)-O-(C₁-C₄)-alkyl, 3-hydroxypyrrole-2,4-diones, 5-oxo-1,2,4-thiadiazoles, quinazoline, quinoxaline, purine, indole, pteridine, tetrahydroquinoline, tetrahydroisoquinoline and isoquinoline, and
R¹, R², and R⁴ in each case are hydrogen,
R⁵ is hydrogen and
R⁶ is
1. phenyl, mono- or disubstituted, independently of one another, by
1.1 -CN,
1.2 -CF₃,
1.3 halogen,
1.4 -O-R¹⁰,
1.5 -N(R¹⁰)₂,
1.6 -NH-C (O) -R¹¹,
1.7 -S(O)x -R¹⁰, where x is the integer zero, 1 or 2,
1.8 -C(O)-R¹¹ or
1.9 - (C₁-C₄) -alkyl-NH₂ ,
2. heteroaryl having 5 to 14 ring members, where heteroaryl is as defined above and is unsubstituted or mono-, di- or trisubstituted, independently of one another, by the substituents defined above under 1.1 to 1.9 or
3. heterocycle having 5 to 12 ring members, where heterocycle is as defined above and is unsubstituted or mono-, di- or trisubstituted, independently of one another, by the substituents defined above under 1.1 to 1.9.

2. A compound of the formula I as claimed in claim 1, wherein
R³ is a radical of the formula II, in which
D is -C(O) -,
R⁷ is hydrogen,
Z is -C (O) -OH or -C(O)-NH₂,
R⁸ is
1. -(C₁-C₄)-alkyl, where alkyl is straight-chain or branched and is mono- or disubstituted, independently of one another, by
1.1 -S(O)-R¹⁰, where R¹⁰ is as defined below,
1.2 -N(R¹⁰)₂, where R¹⁰ is as defined below, or
1.3 pyrrole or
2. is the characteristic radical of an amino acid from the group consisting of histidine, tryptophan, serine, threonine, cysteine, methionine, asparagine, glutamine, lysine, arginine, glutamic acid and aspartic acid,
R⁹ is
1. hydrogen,
2. - (C₁-C₄) -alkyl, where alkyl is straight-chain or branched and, independently of one another, mono-, di- or trisubstituted by -C (O) -OH, -OH or -C(O)-NH₂, or
3. phenyl, where phenyl is unsubstituted or mono- to trisubstituted, independently of one another, by halogen or -(C₁-C₄)-alkyl,
R¹⁰ is
a) hydrogen,
b) - (C₁-C₆) -alkyl, where alkyl is unsubstituted or mono- to trisubstituted, independently of one another, by halogen,
c) phenyl, where phenyl is unsubstituted or mono- to trisubstituted, independently of one another, by halogen or -(C₁-C₄)-alkyl,
R¹, R² and R⁴ in each case are hydrogen,
R⁵ is hydrogen, and
R⁶ is phenyl or pyridine.

3. A process for preparing the compound of the formula I as claimed in claims 1 or 2, which comprises
a) reacting a compound of the formula IV, 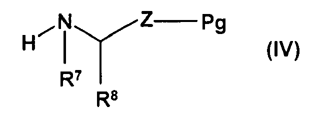 in which Pg is a suitable protective group (for example methyl ester), an amide group or a hydroxyl group and Z, R⁷ and R⁸ are as defined in formula I, 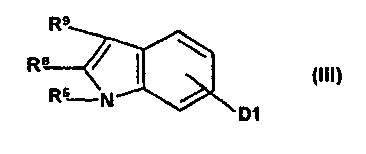 with an acyl chloride or an activated ester of the compound of the formula III,
where D1 is -COOH or sulfonyl halogen and R⁵, R⁶ and R⁹ are as defined in formula I, in the presence of a base or, if appropriate, of a dehydrating agent in solution and, after removal of the protective group, converting into a compound of the formula I, or
b) reacting a compound of the formula IVa, 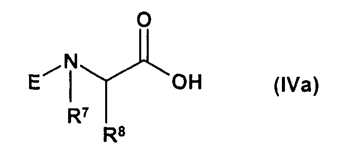 in which R⁷ and R⁸ are as defined in formula I and E is an N-amino protective group, with its carbonyl group coupled via an intermediate chain L to a polymeric resin of the formula PS, a compound of the formula V 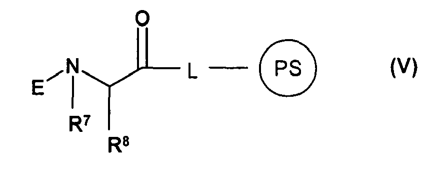 resulting, which, after selective removal of the protective group E, is reacted with a compound of the formula III, where R⁵, R⁶ and R⁹ are as defined in formula I, in the presence of a base or, if appropriate, of a dehydrating agent to give a compound of the formula VI 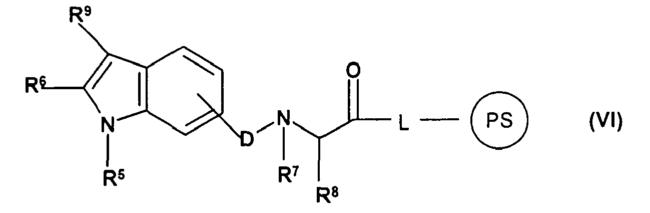 and converting the compound of the formula VI, after cleavage from the support material, into a compound of the formula I, or
c) converting a compound of the formula I into a physiologically acceptable salt.

4. A pharmaceutical comprising an efficacious amount of at least one compound of the formula I as claimed in claims 1 or 2 together with a pharmaceutically suitable and physiologically acceptable excipient, additive and/or other active compounds and auxiliaries.

5. The use of at least one compound of the formula I as claimed in claims 1 or 2 for preparing pharmaceuticals for the prophylaxis and therapy of disorders in the course of which an increased activity of NFκB is involved.

6. The use as claimed in claim 5 for the treatment of chronic disorders of the locomotor apparatus, such as inflammatory, immunological or metabolic acute and chronic arthritic disorders, arthropathies, rheumatoid arthritis, or degenerative joint disorders, such as osteoarthroses, spondyloses, cartilage breakdown following joint trauma or prolonged immobilization of a joint after meniscus or patella injuries or desmorrhexis or disorders of the connective tissue, such as collagenoses and periodontal disorders, myalgias and disturbances of the bone metabolism, or disorders caused by overexpression of tumor necrosis factor alpha (TNFα) or increased concentration of TNFα, such as cachexia, multiple sclerosis, skull-brain trauma, Crohn's disease and intestinal tumors, or atherosclerosis, stenoses, ulceration, Alzheimer's disease, muscle wasting, carcinomatous disorders (potentiation of therapies with cytotoxic compounds), myocardial infarction, gout, sepsis, septic shock, endotoxic shock, viral infections, such as flu, hepatitis, HIV infections, AIDS, or disorders caused by adenoviruses or herpes viruses, parasitic infections, such as malaria or leprosy, fungal infections or yeast infections, meningitis, chronic inflammatory lung diseases, such as chronic bronchitis or asthma, acute respiratory distress syndrome, acute synovitis, tuberculosis, psoriasis, diabetes, treatment of acute or chronic rejection responses of the organ recipient to the transplanted organ, chronic graft-versus-host disorders and inflammatory vascular disorders.

7. A process for preparing a pharmaceutical, which comprises bringing at least one compound of the formula I as claimed in claims 1 or 2 into a suitable administration form using a pharmaceutically suitable and physiologically acceptable excipient and, if appropriate, further suitable active compounds, additives or auxiliaries.

## Revendications

1. Composé de formule I 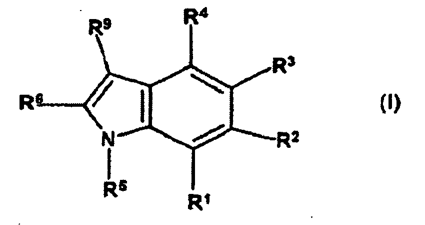 et/ou une forme stéréo-isomère du composé de formule I et/ou un sel physiologiquement acceptable du composé de formule I, où
R³ représente un radical de formule II 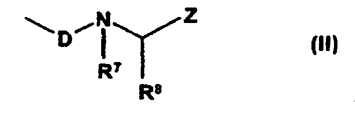
où
D représente -C(O)-,
R⁷ représente un atome d'hydrogène ou -(C₁-C₄)-alkyle,
R⁸ représente
1. - (C₁-C₄) -alkyle, où alkyle est linéaire ou ramifié et est monosubstitué ou disubstitué, indépendamment l'un de l'autre, par
1.1 hétéroaryle comprenant 5 à 14 chaînons de cycle ou hétérocycle comprenant 5 à 12 chaînons de cycle, où hétéroaryle et hétérocycle ont été choisis dans le groupe constitué par pyrrole, pyridine, pyrazine, furanne, thiophène, imidazole, pyrazole, oxazole, isoxazole, thiazole, isothiazole, tétrazole, triazolones, 1,2,3,5-oxathiadiazol-2-oxydes, oxadiazolones, isoxazolones, oxadiazolidinediones, triazoles, qui sont substitués par F, -CN, -CF₃ ou -C(O)-O-(C₁-C₄)-alkyle, 3-hydroxypyrro-2,4-diones, 5-oxo-1,2,4-thiadiazoles, pyrimidine, indole, iso-indole, indazole, phtalazine, quinoléine, isoquinoléine, quinoxaline, quinazoline, cinnoline, carboline et des dérivés benzannellés, cyclopenta-annelés, cyclohexa-annelés et cyclohepta-annelés de ces hétérocycles,
1.2 -O-R¹⁰,
1.3 -S(O)ₓ-R¹⁰, où x représente le nombre entier zéro, 1 ou 2,
1.4 -N(R¹⁰)₂,
1.5 un radical de formule 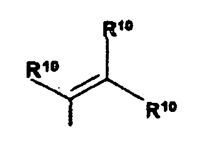 ou
1.6 un radical de formule 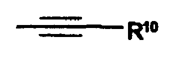
ou
2. le reste caractéristique d'un acide aminé du groupe formé par la glycine, l'alanine, la valine, la leucine, l'isoleucine, la phénylalanine, la tyrosine, la sérine, le tryptophane, la thréonine, la cystéine, la méthionine, l'asparagine, la glutamine, la lysine, l'histidine, l'arginine, l'acide glutamique, l'acide aspartique, l'acide 2-aminoadipique, l'acide 2-amino-isobutyrique, l'acide 2-aminobutyrique, l'acide 2,3-diaminopropionique, l'acide 2,4-diaminobutyrique, l'acide 1,2,3,4-tétrahydro-isoquinoléine-1-carboxylique, l'acide 1,2,3,4-tétrahydro-isoquinoléine-3-carboxylique, l'acide 2-aminopimélique, la phénylglycine, la 3-(2-thiényl)-alanine, la 3-(3-thiényl)-alanine, la 2-(2-thiényl)-glycine, l'acide 2-aminoheptanoïque, l'acide pipécolique, l'hydroxylysine, la sarcosine, la N-méthylisoleucine, la 6-N-méthyllysine, la N-méthylvaline, la norvaline, la norleucine, l'ornithine, l'allo-isoleucine, l'allo-thréonine, l'allo-hydroxylysine, la 4-hydroxyproline, la 3-hydroxyproline, la 3-(2-naphtyl)-alanine, la 3-(1-naphtylalanine), l'homophénylalanine, l'homocystéine, l'acide homocystéique, l'homotryptophane, l'acide cystéique, la 3-(2-pyridyl)-alanine, la 3-(3-pyridyl)-alanine, la 3-(4-pyridyl)-alanine, l'acide 2-amino-3-phénylaminopropionique, l'acide 2-amino-3-phénylaminoéthylpropionique, la phosphinothricine, la 4-fluorophénylalanine, la 3-fluorophénylalanine, la 2-fluorophénylalanine, la 4-chlorophénylalanine, la 4-nitrophénylalanine, la 4-aminophénylalanine, la citrulline, la cyclohexylalanine, le 5-fluorotryptophane, le 5-méthoxytryptophane, la méthionine-sulfone, le méthionine-sulfoxyde et -NH-NR¹⁰-C(O)N(R¹⁰)₂,
R⁹ représente
1. R⁸,
2. -(C₁-C₄)-alkyle, où alkyle est linéaire ou ramifié et est monosubstitué, disubstitué ou trisubstitué, indépendamment l'un de l'autre, par
2.1 aryle, où aryle représente un radical du groupe phényle, naphtyle, biphénylyle, anthryle ou fluorényle et le radical aryle est non substitué ou monosubstitué, disubstitué ou trisubstitué par des radicaux identiques ou différents de la série - (C₁-C₈)-alkyle, - (C₁-C₈) -alcoxy, halogène, nitro, amino, trifluorométhyle, hydroxy, hydroxy- (C₁-C₄) -alkyle tel qu'hydroxyméthyle ou 1-hydroxyéthyle ou 2-hydroxyéthyle, méthylènedioxy, éthylènedioxy, formyle, acétyle, cyano, hydroxycarbonyle, aminocarbonyle, -(C₁-C₄)-alcoxycarbonyle, phényle, phénoxy, benzyle, benzyloxy ou tétrazolyle,
2.2 halogène,
2.3 -CN ou
2.4 -CF₃,
3. aryle, où aryle est tel que défini ci-dessus et est non substitué ou substitué comme ci-dessus,
R¹⁰ représente
a) un atome d'hydrogène
b) - (C₁-C₆) -alkyle, où alkyle est non substitué ou monosubstitué à trisubstitué, indépendamment l'un de l'autre, par
1. aryle, où aryle est défini comme ci-dessus,
2. hétéroaryle comprenant 5 à 14 chaînons de cycle, où hétéroaryle est défini comme ci-dessus,
3. hétérocycle comprenant 5 à 12 chaînons de cycle, où hétérocycle est défini comme ci-dessus,
4. halogène,
5. -N- (C₁-C₆)ₙ-alkyle, où n signifie le nombre entier zéro, 1 ou 2 et alkyle est non substitué ou monosubstitué, disubstitué ou trisubstitué, indépendamment l'un de l'autre, par halogène ou par -C(O)-OH, ou
6. -C(O)-OH,
c) aryle, où aryle est défini comme ci-dessus,
d) hétéroaryle comprenant 5 à 14 chaînons de cycle, où hétéroaryle est défini comme ci-dessus, ou
e) hétérocycle comprenant 5 à 12 chaînons de cycle, où hétérocycle est défini comme ci-dessus, et
dans le cas de (R¹⁰) ₂, R¹⁰ représente, indépendamment l'un de l'autre, la signification de a) à e),
Z représente
1. 1,3,4-oxadiazole, où 1,3,4-oxadiazole est non substitué ou monosubstitué à trisubstitué par -NH₂, OH ou - (C₁-C₄) -alkyle ou
2. -C (O) -R¹¹, où
R¹¹ représente
1. -O-R¹⁰ ou
2. -N(R¹⁰)₂, ou
R⁷ et R⁸ forment ensemble avec l'atome d'azote et de carbone auxquels ils sont à chaque fois liés un cycle de formule IIa 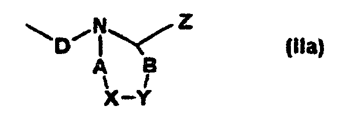 du groupe pyrrole, pyrroline, pyrrolidine, pyridine, pipéridine, pipérylène, pyridazine, pyrimidine, pyrazine, pipérazine, pyrazole, imidazole, pyrazoline, imidazoline, pyrazolidine, imidazolidine, oxazole, tétrazole, 1,2,3,5-oxathiadiazol-2-oxydes, triazolones, oxadiazolones, isoxazolones, oxadiazolidinediones, triazoles, qui sont non substitués ou substitués par F, -CN, -CF₃ ou C(O)-O-(C₁-C₄)-alkyle, 3-hydroxypyrro-2,4-diones, 5-oxo-1,2,4-thiadiazoles, isoxazole, 2-isoxazolidine, isoxazolidine, morpholine, isothiazole, thiazole, isothiazolidine, thiomorpholine, indazole, thiadiazole, benzimidazole, quinoléine, triazole, phtalazine, quinazoline, quinoxaline, purine, ptéridine, indole, isoquinoléine, tétrahydroquinoléine et tétrahydro-isoquinoléine, ou
R⁸ et Z forment ensemble avec les atomes de carbone auxquels ils sont à chaque fois liés un cycle de formule IIc 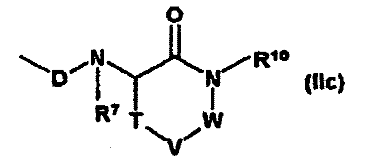 du groupe pyrrole, pyrroline, pyrrolidine, pyridine, pipéridine, pyrazoline, phtalazine, pipérylène, pyridazine, pyrimidine, pyrazine, pipérazine, pyrazole, imidazole, 1,3,4-oxadiazole, imidazoline, pyrazolidine, imidazolidine, oxazole, isoxazole, 2-isoxazolidine, isoxazolidine, morpholine, isothiazole, thiazole, isothiazolidine, thiomorpholine, indazole, thiadiazole, benzimidazole, quinoléine, triazole, tétrazole, 1,2,3,5-oxathiadiazol-2-oxydes, oxadiazolones, isoxazolones, triazolones, oxadiazolidinediones, triazoles, qui sont non substitués ou substitués par F, -CN, -CF₃ ou -C(O)-O-(C₁-C₄)-alkyle, 3-hydroxypyrro-2,4-diones, 5-oxo-1,2,4-thiadiazoles, quinazoline, quinoxaline, purine, indole, ptéridine, tétrahydroquinoléine, tétrahydro-isoquinoléine et isoquinoléine, et
R¹, R² et R⁴ représentent un atome d'hydrogène,
R⁵ représente un atome d'hydrogène, et
R⁶ représente
1. phényle, monosubstitué ou disubstitué, indépendamment l'un de l'autre, par
1.1 -CN
1.2 -CF₃,
1.3 halogène,
1.4 -O-R¹⁰,
1. 5 -N(R¹⁰) ₂,
1.6 -NH-C(O) -R¹¹,
1.7 -S(O)ₓ-R¹⁰, où x représente le nombre entier zéro, 1 ou 2,
1. 8 -C (O) -R¹¹ ou
1.9 - (C₁-C₄) -alkyl-NH₂,
2. hétéroaryle comprenant 5 à 14 chaînons de cycle, où hétéroaryle est défini comme ci-dessus et est non substitué ou monosubstitué, disubstitué ou trisubstitué, indépendamment l'un de l'autre, par les substituants définis ci-dessus aux points 1.1 à 1.9 ou
3. hétérocycle comprenant 5 à 12 chaînons de cycle, où hétérocycle est défini comme ci-dessus et est non substitué ou monosubstitué, disubstitué ou trisubstitué, indépendamment l'un de l'autre, par les substituants définis ci-dessus aux points 1.1 à 1.9.

2. Composé de formule I, selon la revendication 1, **caractérisé en ce que**
R³ représente un radical de formule II, où
D représente -C(O)-,
R⁷ représente un atome d'hydrogène,
Z représente -C (O) -OH ou -C (O) -NH₂,
R⁸ représente
1. -(C₁-C₄)-alkyle, où alkyle est linéaire ou ramifié et est monosubstitué ou disubstitué, indépendamment l'un de l'autre, par
1.1 -S(O)-R¹⁰, où R¹⁰ est défini comme ci-dessous,
1.2 -N(R¹⁰) ₂, où R¹⁰ est défini comme ci-dessous, ou
1.3 pyrrole, ou
2. le reste caractéristique d'un acide aminé du groupe histidine, tryptophane, sérine, thréonine, cystéine, méthionine, asparagine, glutamine, lysine, arginine, acide glutamique et acide aspartique,
R⁹ représente
1. un atome d'hydrogène
2. -(C₁-C₄)-alkyle, où alkyle est linéaire ou ramifié et est monosubstitué, disubstitué ou trisubstitué, indépendamment l'un de l'autre, par -C(O)-OH, -OH ou -C(O)-NH₂, ou
3. phényle, où phényle est non substitué ou monosubstitué à trisubstitué, indépendamment l'un de l'autre, par halogène ou -(C₁-C₄)-alkyle,
R¹⁰ représente
a) un atome d'hydrogène
b) -(C₁-C₆)-alkyle, où alkyle est non substitué ou monosubstitué à trisubstitué, indépendamment l'un de l'autre, par halogène,
c) phényle, où phényle est non substitué ou monosubstitué à trisubstitué, indépendamment l'un de l'autre, par halogène ou -(C₁-C₄)-alkyle,
R¹, R² et R⁴ représentent un atome d'hydrogène,
R⁵ représente un atome d'hydrogène, et
R⁶ représente phényle ou pyridine.

3. Procédé pour la préparation du composé de formule I selon les revendications 1 ou 2, **caractérisé en ce qu'**on
a) transforme un composé de formule IV, 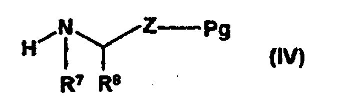 où Pg représente un groupe de protection approprié (par exemple un ester de méthyle), un groupe amide ou un groupe hydroxy et Z, R⁷ et R³ sont définis comme dans la formule I, avec 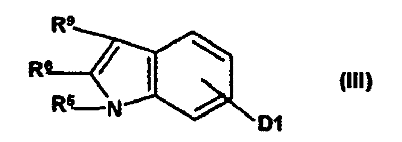 un chlorure d'acide ou un ester activé du composé de formule III, où D1 signifie -COOH ou sulfonylhalogène et R⁵, R⁶ et R⁹ sont définis comme dans la formule I, en présence d'une base ou le cas échéant d'un agent éliminant l'eau, en solution et on transforme, après dissociation du groupe de protection en un composé de formule I, ou
b) couple un composé de formule IVa, 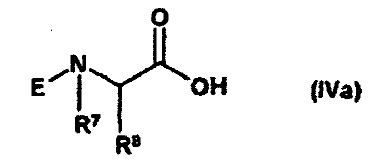 où R⁷ et R⁸ sont tels que définis dans la formule I et E représente un groupe de protection N-amino, avec son groupe carboxyle via une chaîne intermédiaire L sur une résine polymère de formule générale PS, avec formation d'un composé de formule V 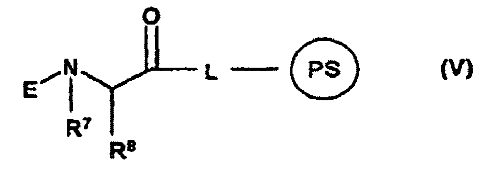 qui est transformé, après dissociation sélective du groupe de protection E avec un composé de formule III, où R⁵, R⁶ et R⁹ sont définis comme dans la formule I, en présence d'une base ou le cas échéant d'un agent éliminant l'eau, en un composé de formule VI 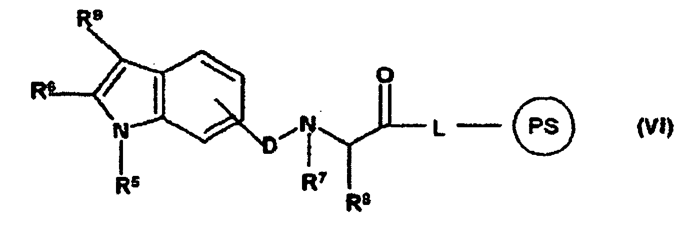 et on transforme le composé de formule VI après dissociation du matériau support en un composé de formule I, ou
c) transforme un composé de formule I en un sel physiologiquement acceptable.

4. Médicament, **caractérisé par** une teneur active en au moins un composé de formule I selon les revendications 1 ou 2 avec un support, un additif pharmaceutiquement approprié et physiologiquement compatible et/ou d'autres substances actives et adjuvants.

5. Utilisation d'au moins un composé de formule I selon les revendications 1 ou 2 pour la préparation de médicaments destinés à la prophylaxie et la thérapie de maladies à l'évolution desquelles participe une activité renforcée de NFκB.

6. Utilisation selon la revendication 5 pour le traitement de maladies chroniques de l'appareil locomoteur, telles que les arthrites inflammatoires, immunologiques ou métaboliques, aiguës ou chroniques, les arthropathies, la polyarthrite rhumatoïde, ou les maladies de dégénérescence des articulations, telles que les ostéoarthroses, les spondyloses, l'atrophie du cartilage après un traumatisme des articulations ou un repos prolongé des articulations après des lésions au ménisque ou à la rotule ou des déchirures de ligament ou des maladies du tissu conjonctif, telles que les collagénoses et les maladies périodontiques, les myalgies et les troubles du métabolisme osseux ou les maladies qui sont provoquées par une surexpression du facteur de nécrose tumoral alpha (TNFα) ou par une concentration élevée en TNFα, telles que la cachexie, la sclérose en plaques, un traumatisme au crâne-cerveau, la maladie de Crohn et les ulcères intestinaux, ou l'athérosclérose, les sténoses, l'ulcération, la maladie d'Alzheimer, une détérioration musculaire, les maladies cancéreuses (augmentation de la puissance de thérapies cytotoxiques), l'infarctus cardiaque, la goutte, une septicémie, un choc septique, un choc endotoxique, les infections virales telles que la grippe, l'hépatite, les infections au HIV, le SIDA, ou les maladies provoquées par des adénovirus ou les virus de l'herpes, les infections parasitaires, telles que la malaria ou la lèpre, les infections par des champignons ou des levures, les inflammations des méninges, les maladies chroniques inflammatoires des poumons, telles que la bronchite chronique ou l'asthme, le syndrome de détresse respiratoire aigu, la synovite aiguë, la tuberculose, le psoriasis, le diabète, le traitement de maladies de rejet aiguës ou chroniques de l'organisme receveur contre l'organe transplanté, les maladies chroniques greffe-contre-hôte et les maladies vasculaires inflammatoires.

7. Procédé pour la préparation d'un médicament, **caractérisé en ce qu'**on met sous une forme d'administration appropriée au moins un composé de formule I selon les revendications 1 ou 2 avec un support pharmaceutiquement approprié et physiologiquement acceptable et le cas échéant d'autres substances actives, additifs ou adjuvants appropriés.
